# EUROPÄISCHE PATENTSCHRIFT

(11) **EP 3 029 054 B1**
(45) Veröffentlichungstag und Bekanntmachung des Hinweises auf die Patenterteilung: **23.11.2016**
(21) Anmeldenummer: 14196196.1
(22) Anmeldetag: 04.12.2014
(51) Int. Cl.: C07F 9/6574, C07B 41/06, C07C 45/50

(54) **Phosphoramidite welche eine Phenyl-Phenyl-Einheit oder eine Phenyl-Naphthyl-Einheit aufweisen**
Phosphoramidites containing a phenyl-phenyl unit or a phenyl-naphthyl unit
Phosphoramidite présentant une unité de phényle-phényle ou une unité de phényle-naphtyle

(43) Veröffentlichungstag der Anmeldung: 08.06.2016
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Hess, Dieter, 45770 Marl (DE); Fridag, Dirk, 45721 Haltern am See (DE); Geilen, Frank, 45721 Haltern am See (DE); Börner, Armin, 18059 Rostock (DE); Selent, Detlef, 18059 Rostock (DE)

(56) Entgegenhaltungen:
- WO-A2-2004/076464
- Annemiek Van Rooy ET AL: "Phosphoramidites: novel modifying ligands in rhodium catalysed hydro formy lation", , 1996, XP055133271, Gefunden im Internet: URL:http://onlinelibrary.wiley.com/store/1 0.1002/recl.19961151108/asset/19961151108_ ftp.pdf?v=1&t=hyh5iz2i&s=9c2e6e1133731ef17 1f9525516c98c7635860bdc [gefunden am 2014-08-05]

## Beschreibung

Die Erfindung betrifft Phosphoramidite, welche eine Phenyl-Phenyl-Einheit oder eine Phenyl-Naphthyl-Einheit aufweisen. Des Weiteren ein Verfahren zu deren Herstellung, sowie die weitere Umsetzung zu Monophosphiten, welche als Liganden in der Hydroformylierung verwendet werden können. Die Phosphoramidite selbst können ebenfalls als Liganden eingesetzt werden.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor P(III). Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Beispielsweise ist die Verbindung 6-(Naphthalin-1-yloxy)dibenzo[d,f][1,3,2]dioxaphosphepin ist bereits bekannt (CAS 16611-80-6) und kann durch die Reaktion von 1-Naphthol und 6-Chlorodibenzo[*d,f*][1,3,2]dioxaphosphepin in Gegenwart einer Base hergestellt werden.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Phosphoramidite, d.h. Verbindungen, die anstelle der P-O über eine oder mehrere P-N-Bindungen verfügen, wurden bisher nur selten als Liganden in der Hydroformylierung eingesetzt.

Phosphoramidite, welche eine Phenyl-Phenyl-Einheit aufweisen sind in der Literatur bereits beschrieben:
- Javier Mazuela et al., Tetrahedron: Asymmetry 21 (2010) 2153-2157; Seite 2154, Verbindung L2;
- Van Leeuwen und Mitarbeiter (A. van Rooy, D. Burgers, P. C. J. Kamer, P. W. N. M. van Leeuwen, Recl. Trav. Chim. Pays-Bas 1996, 115, 492) untersuchten erstmals monodentate Phosphoramidite in der Hydroformylierung. Insgesamt wurden bei den hohen bis extrem hohen Ligand / Rhodium-Verhältnissen von bis zu 1000 : 1 nur mäßige katalytische Eigenschaften beobachtet.

Weiterhin sind Phosphoramidite als Liganden für Hydroformylierungskatalysatoren aus WO2004/076464 sowie durch Annemiek van Rooy et al. in Recl. Trav. chim. Pays-Bas 1996, 115, Seiten 492-498 benannt.

Die in der Literatur beschriebenen Biphenol-Einheiten sind symmetrisch. Das bedeutet, dass man zwischen die beiden Phenylringe eine Spiegelebene legen kann, so dass der eine auf den anderen abgebildet werden kann.

Der Erfindung lag die Aufgabe zugrunde, Phosphoramidite bereitzustellen, welche gegenüber den bekannten Phosphoramidite vorteilhafte Eigenschaften aufweisen. Die Phosphoramidite sollen sich zur Synthese von Monophosphiten eignen, welche in der Hydroformylierung von Olefinen eingesetzt werden können. Mit den so erhaltenen Monophosphiten sollen guten Ergebnisse in der Hydroformylierung (insbesondere gute Selektivitäten) erzielt werden.

Die Aufgabe wird gelöst durch eine Verbindung nach Anspruch 1.

Verbindung, welche eine der allgemeinen Strukturen (**I**) oder (**II**) aufweist: 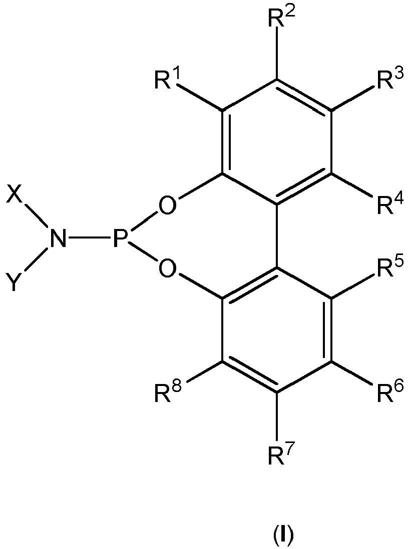 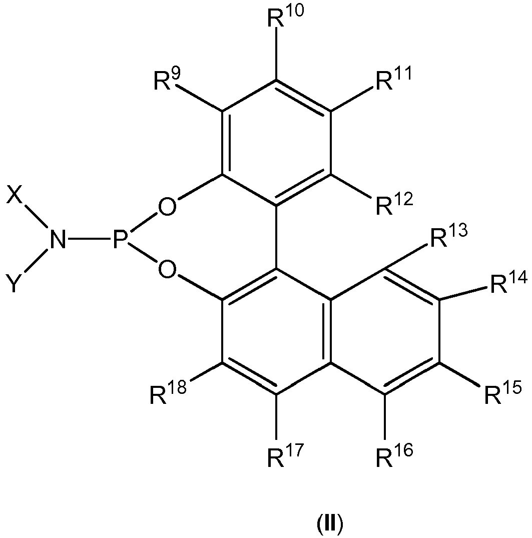 wobei
R¹, R², R³, R⁴, R⁵, R⁶, R⁷, R⁸ ausgewählt sind aus:
   - H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R⁹, R¹⁰, R¹¹, R¹², R¹³, R¹⁴, R¹⁵, R¹⁶, R¹⁷, R¹⁸ ausgewählt sind aus:
   -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
   wobei die genannten Alkyl- und Arylgruppen substituiert sein können,
und X und Y ausgewählt sind aus:
   -(C₁-C₁₂)-Alkyl, -(C₃-C₁₂)-Cycloalkyl, -(C₃-C₁₂)-Heterocycloalkyl, -(C₆-C₂₀)-Aryl,
   wobei die genannten Alkyl- und Arylgruppen substituiert sein können,
   und die beiden Reste mindestens eines der vier folgenden Restepaare nicht für den gleichen Rest stehen: R¹ und R⁸, R² und R⁷, R³ und R⁶, R⁴ und R⁵.

Durch das Merkmal "und die beiden Reste mindestens eines der vier folgenden Restepaare nicht für den gleichen Rest stehen: R¹ und R⁸, R² und R⁷, R³ und R⁶, R⁴ und R⁵" wird zum Ausdruck gebracht, dass es sich hierbei um ein unsymmetrisches Biaryl handelt. Die beiden Aromaten lassen sich nicht durch eine zwischen ihnen liegende Spiegelebene aufeinander spiegeln.

Zugelassen sind folgende Restepaarungen, wie beispielsweise:
R¹ ungleich R⁸, R² gleich R⁷, R³ gleich R⁶, R⁴ gleich R⁵;
R¹ gleich R⁸, R² gleich R⁷, R³ ungleich R⁶, R⁴ gleich R⁵.

Ober aber auch Restepaarungen bei denen mehr als nur ein Paar ungleich ist, wie beispielsweise:
R¹ ungleich R⁸, R² gleich R⁷, R³ ungleich R⁶, R⁴ gleich R⁵;
R¹ ungleich R⁸, R² ungleich R⁷, R³ ungleich R⁶, R⁴ gleich R⁵.

Ausgeschlossen wird lediglich der Fall, bei dem alle vier Restepaare jeweils paarweise für den gleichen Rest stehen:
R¹ gleich R⁸, R² gleich R⁷, R³ gleich R⁶, R⁴ gleich R⁵.
Hierbei würde es sich um ein symmetrisches Biaryl handeln.

(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.

(C₆-C₂₀)-Aryl und -(C₆-C₂₀)-Aryl-(C₆-C₂₀)-Aryl- können jeweils unsubstituiert oder durch einen oder mehrere gleiche oder verschiedene Reste substituiert sein, die ausgewählt sind unter -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Dimethylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, -Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.

Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -(C₃-C₁₂)-Heterocycloalkylgruppen Tetrahydrothiophenyl, Tetrhydrofuryl, Tetrahydropyranyl und Dioxanyl.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter - H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I),-COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂. Substituierte -(C₆-C₂₀)-Arylgruppen und -(C₆-C₂₀)-Aryl-(C₆-C₂₀)-Arylgruppen sind vorzugsweise substituierte -(C₆-C₁₀)-Arylgruppen und -(C₆-C₁₀)-Aryl-(C₆-C₁₀)-Arylgruppen, insbesondere substituiertes Phenyl oder substituiertes Naphthyl oder substituiertes Anthracenyl. Substituierte -(C₆-C₂₀)-Arylgruppen tragen vorzugsweise eine oder mehrere z.B. 1, 2, 3, 4 oder 5 Substituenten, ausgewählt unter -(C₁-C₁₂)-Alkylgruppen, -(C₁-C₁₂)-Alkoxygruppen.

In einer Ausführungsform sind X und Y ausgewählt aus:
-(C₁-C₁₂)-Alkyl, -(C₃-C₁₂)-Cycloalkyl, -(C₆-C₂₀)-Aryl.

In einer Ausführungsform sind X und Y ausgewählt aus:
-(C₁-C₁₂)-Alkyl, -(C₃-C₁₂)-Cycloalkyl.

In einer Ausführungsform stehen X und Y für:
-(C₁-C₁₂)-Alkyl.

In einer Ausführungsform stehen X und Y für den gleichen Rest.

In einer weiteren Ausführungsform stehen X und Y für unterschiedliche Reste.

In einer Ausführungsform sind R¹, R², R³, R⁴, R⁵, R⁶, R⁷, R⁸ ausgewählt aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

In einer Ausführungsform sind R⁹, R¹⁰, R¹¹, R¹², R¹³, R¹⁴, R¹⁵, R¹⁶, R¹⁷, R¹⁸ ausgewählt aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

In einer Ausführungsform sind R¹, R², R³, R⁴, R⁵, R⁶, R⁷, R⁸ ausgewählt aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen.

In einer Ausführungsform sind R⁹, R¹⁰, R¹¹, R¹², R¹³, R¹⁴, R¹⁵, R¹⁶, R¹⁷, R¹⁸ ausgewählt aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen.

In einer Ausführungsform sind R¹, R², R³, R⁴, R⁵, R⁶, R⁷, R⁸ ausgewählt aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

In einer Ausführungsform sind R⁹, R¹⁰, R¹¹, R¹², R¹³, R¹⁴, R¹⁵, R¹⁶, R¹⁷, R¹⁸ ausgewählt aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

In einer Ausführungsform sind R¹, R², R³, R⁴, R⁵, R⁶, R⁷, R⁸ ausgewählt aus:
-H, -(C₁-C₁₂)-Alkyl, -O-(C₁-C₁₂)-Alkyl, -O-(C₆-C₂₀)-Aryl.

In einer Ausführungsform sind R⁹, R¹⁰, R¹¹, R¹², R¹³, R¹⁴, R¹⁵, R¹⁶, R¹⁷, R¹⁸ ausgewählt aus:
-H, -(C₁-C₁₂)-Alkyl, -O-(C₁-C₁₂)-Alkyl, -O-(C₆-C₂₀)-Aryl.

In einer Ausführungsform sind R¹, R², R³, R⁴, R⁵, R⁶, R⁷, R⁸ ausgewählt aus:
-H, -(C₁-C₁₂)-Alkyl, -O-(C₁-C₁₂)-Alkyl.

In einer Ausführungsform sind R⁹, R¹⁰, R¹¹, R¹², R¹³, R¹⁴, R¹⁵, R¹⁶, R¹⁷, R¹⁸ ausgewählt aus:
-H, -(C₁-C₁₂)-Alkyl, -O-(C₁-C₁₂)-Alkyl.

In einer Ausführungsform weist die Verbindung eine der folgenden Strukturen (2') bis (7') auf:

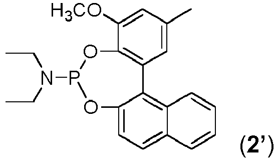

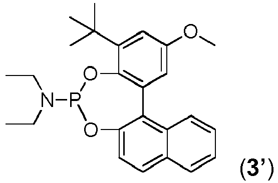

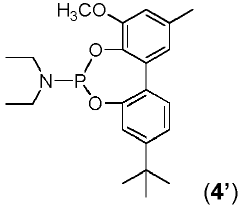

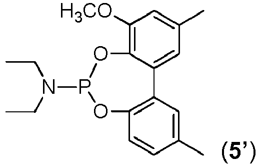

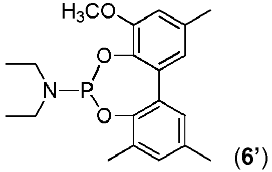

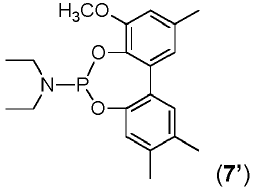

In einer Ausführungsform weist die Verbindung die allgemeinen Strukturen (**I**) auf.

In einer Ausführungsform weist die Verbindung die allgemeinen Strukturen (**II**) auf.

Die beschriebenen Verbindungen können auch Bestandteil eines Ligand-Metall-Komplexes sein, wobei die Verbindung die Liganden darstellen.

Komplex umfassend:
- eine zuvor beschriebene Verbindung,
- ein Metallatom ausgewählt aus: Rh, Ru, Co, Ir.

Vorzugsweise ist das Metall Rh.

Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Solche Ligand-Metall-Komplexe können zur Katalyse einer Hydroformylierungsreaktion verwendet werden.

Verwendung einer zuvor beschriebenen Verbindung in einem Ligand-Metall-Komplex zur Katalyse einer Hydroformylierungsreaktion.

Des Weiteren wird die Verwendung der Verbindung zur Herstellung von Monophosphiten beansprucht.

Verwendung einer zuvor beschriebenen Verbindung zur Herstellung von Monophosphiten.

Verfahren umfassend die Verfahrensschritte:
a) Vorlegen einer Verbindung, welche eine der allgemeinen Strukturen (**III**) oder (**IV**) aufweist: 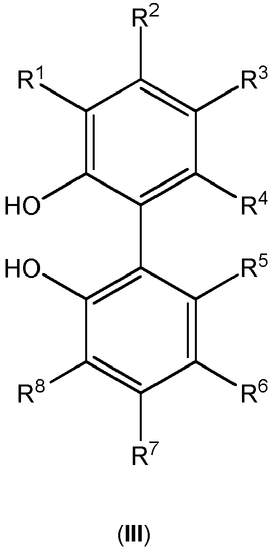 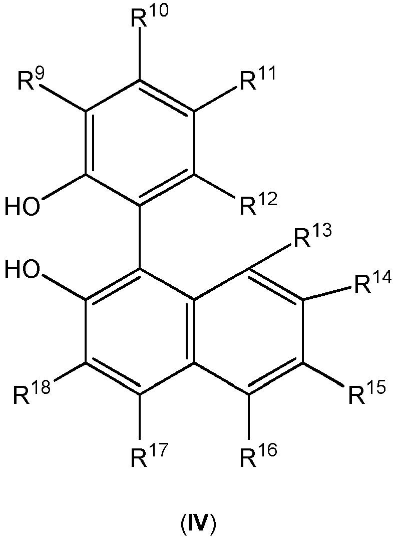 wobei
   R¹, R², R³, R⁴, R⁵, R⁶, R⁷, R⁸ ausgewählt sind aus:
      -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
   R⁹, R¹⁰, R¹¹, R¹², R¹³, R¹⁴, R¹⁵ R¹⁶ R¹⁷, R¹⁸ ausgewählt sind aus:
      -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
   wobei die genannten Alkyl- und Arylgruppen substituiert sein können, und die beiden Reste mindestens eines der vier folgenden Restepaare nicht für den gleichen Rest stehen: R¹ und R⁸, R² und R⁷, R³ und R⁶, R⁴ und R⁵;
b) Hinzugeben einer Verbindung, welche die allgemeinen Struktur (**V**) aufweist: 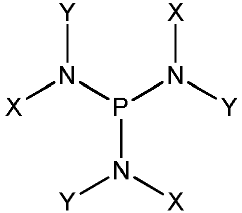 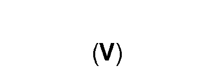 wobei X und Y ausgewählt sind aus:
   -(C₁-C₁₂)-Alkyl, -(C₃-C₁₂)-Cycloalkyl, -(C₃-C₁₂)-Heterocycloalkyl, -(C₆-C₂₀)-Aryl;
c) Durchmischen der Verbindungen aus Verfahrensschritt a) und b) wobei (**V**) mit (**III**) zu (**I**) umgesetzt wird, beziehungsweise (**V**) mit (**IV**) zu (**II**) umgesetzt wird: 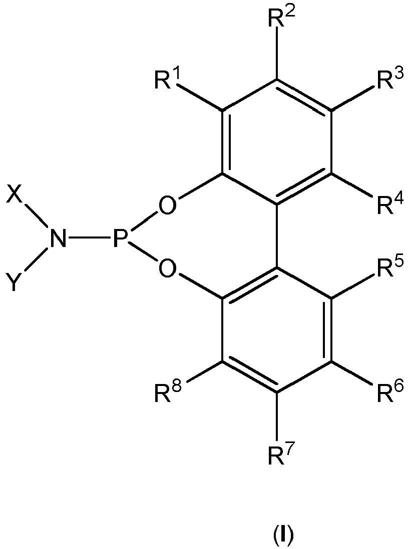 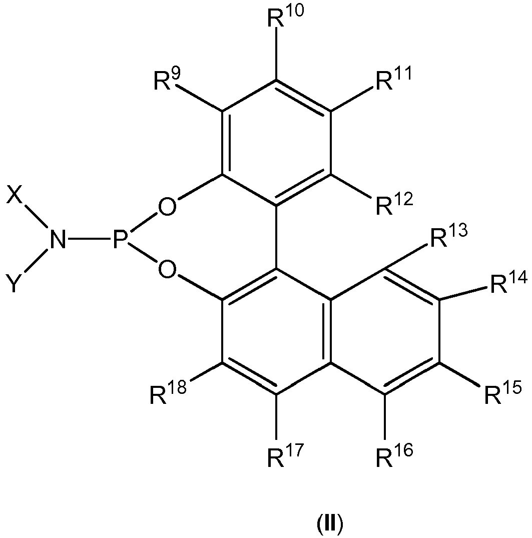 wobei
   R¹, R², R³, R⁴, R⁵, R⁶, R⁷, R⁸ ausgewählt sind aus:
      -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
   R⁹, R¹⁰, R¹¹, R¹², R¹³, R¹⁴, R¹⁵ R¹⁶ R¹⁷, R¹⁸ ausgewählt sind aus:
      -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
      wobei die genannten Alkyl- und Arylgruppen substituiert sein können,
   und X und Y ausgewählt sind aus:
      -(C₁-C₁₂)-Alkyl, -(C₃-C₁₂)-Cycloalkyl, -(C₃-C₁₂)-Heterocycloalkyl, -(C₆-C₂₀)-Aryl,
      wobei die genannten Alkyl- und Arylgruppen substituiert sein können, und die beiden Reste mindestens eines der vier folgenden Restepaare nicht für den gleichen Rest stehen: R¹ und R⁸, R² und R⁷, R³ und R⁶, R⁴ und R⁵.

Hierbei können die Verfahrensschritte a) und b) auch in umgekehrter Reihenfolge erfolgen.

In einer Variante des Verfahrens umfasst diese den zusätzlichen Verfahrensschritt: d) Zugabe von Naphthol zu dem Reaktionsprodukt aus Verfahrensschritt c).

In einer Variante des Verfahrens sind X und Y ausgewählt aus:
-(C₁-C₁₂)-Alkyl, -(C₃-C₁₂)-Cycloalkyl, -(C₆-C₂₀)-Aryl.

In einer Variante des Verfahrens sind X und Y ausgewählt aus:
-(C₁-C₁₂)-Alkyl, -(C₃-C₁₂)-Cycloalkyl.

In einer Variante des Verfahrens sind X und Y ausgewählt aus:
-(C₁-C₁₂)-Alkyl.

In einer Variante des Verfahrens stehen X und Y für den gleichen Rest.

In einer weiteren Variante des Verfahrens stehen X und Y für unterschiedliche Reste.

In einer Variante des Verfahrens sind R¹, R², R³, R⁴, R⁵, R⁶, R⁷, R⁸ ausgewählt aus:
- H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

In einer Variante des Verfahrens sind R⁹, R¹⁰, R¹¹, R¹², R¹³, R¹⁴, R¹⁵, R¹⁶, R¹⁷, R¹⁸ ausgewählt aus:
- H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

In einer Variante des Verfahrens sind R¹, R², R³, R⁴, R⁵, R⁶, R⁷, R⁸ ausgewählt aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen.

In einer Variante des Verfahrens sind R⁹, R¹⁰, R¹¹, R¹², R¹³, R¹⁴, R¹⁵, R¹⁶, R¹⁷, R¹⁸ ausgewählt aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen.

In einer Variante des Verfahrens sind R¹, R², R³, R⁴, R⁵, R⁶, R⁷, R⁸ ausgewählt aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

In einer Variante des Verfahrens sind R⁹, R¹⁰, R¹¹, R¹², R¹³, R¹⁴, R¹⁵, R¹⁶, R¹⁷, R¹⁸ ausgewählt aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

In einer Variante des Verfahrens sind R¹, R², R³, R⁴, R⁵, R⁶, R⁷, R⁸ ausgewählt aus:
-H, -(C₁-C₁₂)-Alkyl, -O-(C₁-C₁₂)-Alkyl, -O-(C₆-C₂₀)-Aryl.

In einer Variante des Verfahrens sind R⁹, R¹⁰, R¹¹, R¹², R¹³, R¹⁴, R¹⁵, R¹⁶, R¹⁷, R¹⁸ ausgewählt aus:
-H, -(C₁-C₁₂)-Alkyl, -O-(C₁-C₁₂)-Alkyl, -O-(C₆-C₂₀)-Aryl.

In einer Variante des Verfahrens sind R¹, R², R³, R⁴, R⁵, R⁶, R⁷, R⁸ ausgewählt aus:
-H, -(C₁-C₁₂)-Alkyl, -O-(C₁-C₁₂)-Alkyl.

In einer Variante des Verfahrens sind R⁹, R¹⁰, R¹¹, R¹², R¹³, R¹⁴, R¹⁵, R¹⁶, R¹⁷, R¹⁸ ausgewählt aus:
-H, -(C₁-C₁₂)-Alkyl, -O-(C₁-C₁₂)-Alkyl.

In einer Variante des Verfahrens wird im Verfahrensschritt a) eine Verbindung vorgelegt, welche eine der folgenden Strukturen (**2"**) bis (**7"**) aufweist:

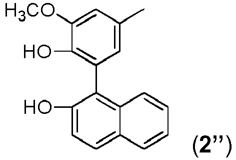

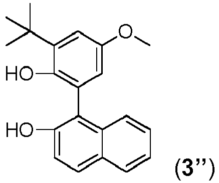

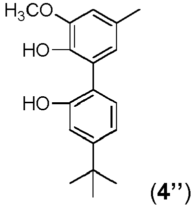

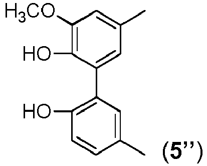

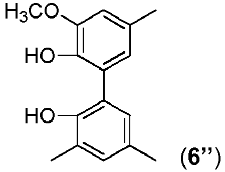

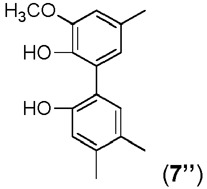

In einer Variante des Verfahrens wird im Verfahrensschritt a) eine Verbindung vorgelegt, welche die allgemeinen Strukturen (**III**) aufweist.

In einer Variante des Verfahrens wird im Verfahrensschritt a) eine Verbindung vorgelegt, welche die allgemeinen Strukturen (**IV**) aufweist.

Im Folgenden wird die Erfindung anhand von Ausführungsbeispielen näher erläutert.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Biphenole

Die Synthese der Biphenole erfolgt analog zu DE102013203865 und DE102013203867.

### Synthese der Chlorophosphite

Die Synthese der Monochlorophosphite wie 6-Chlorodibenzo [d,f][1,3,2]dioxaphosphepin ist dem Fachmann bekannt und erfolgt nach bekannter Weise. Chlorophosphite lassen sich durch Zusatz von Phosphortrichlorit in Gegenwart einer Base herstellen. Für nähere Informationen siehe auch "Phosphorous(III) Ligands in Homogeneous Catalysis - Design and Synthesis" von Paul C.J. Kamer und Piet W.N.M. van Leeuwen; John Wiley and Sons, 2012; unter anderem S. 94 ff. und darin zitierte Literaturstellen.

### Synthese der Monophosphite

### 6-(Naphthalin-1-yloxy)dibenzo[d,f][1,3,2]dioxaphosphepin

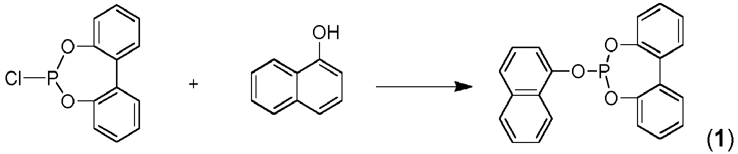

Zu einer auf -20 °C gekühlten Lösung von 1-Naphthol (0,393 g; 2,73 mmol) in THF (8 ml) wird tropfenweise eine Lösung von n-BuLi in Hexan (8,5 ml einer 0,32 M Lösung; 2,73 mmol). Man lässt auf Raumtemperatur erwärmen und gibt zu dieser Mischung eine Lösung von 6-Chlorodibenzo[*d,f*][1,3,2]dioxaphosphepin (0,684 g; 2,73 mmol) in THF (5 ml). Man rührt über Nacht, filtriert und engt das Filtrat zur Trockne ein. Der Rückstand wird durch Säulenchromatografie gereinigt (Hexan/Toluol, 1:1, R*_{f}* = 0,5). Ausbeute: 0,61 g (1,7 mmol; 63%).
³¹P-NMR (CD₂Cl₂): 143,8 ppm.

### 4-Methoxy-2-methyl-6-(naphthalen-1-yloxy)benzo[d]naphtho[1,2-f][1,3,2]dioxaphosphepin

### a) Vorstufe, N,N-Diethyl-2-methoxy-4-methylbenzo[d]naphtho[1,2-f][1,3,2]dioxaphosphepin-6-amin

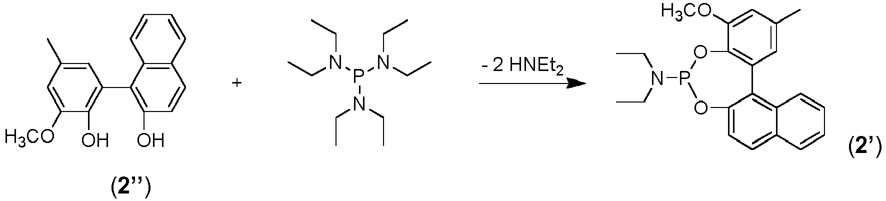

Eine Lösung von 1-(2-Hydroxy-3-methoxy-5-methylphenyl)naphthalen-2-ol (0,661 g; 2,36 mmol) in Toluol (10 ml) wurde mit Tris(diethylamino)phosphin (0,583 g; 2,36 mmol) versetzt. Die Reaktionslösung wurde für 20 h bei Raumtemperatur gerührt. Das Lösungsmittel wurde im Vakuum entfernt und der Rückstand getrocknet. Ausbeute: 0,819 g (2,147 mmol; 90 %). Elementaranalyse (berechnet für C₂₂H₂₄NO₃P = 381,40 g/mol) C 68,69 (68,47); H 6,19 (6,29); N 3,79 (3,80); P 8,45 (8,41)%.
³¹P-NMR (CD₂Cl₂): 149,2; 150,8 ppm.
EI-MS: *m*/*e* 381 [M⁺] (28%); 366 [M-CH₃]⁺ (43%); 309 [M-N(C₂H₅)]⁺ (100%).

### b) 1-Naphthylphosphit

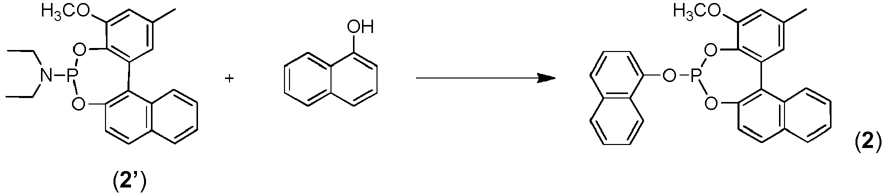

Zu einer gerührten Lösung von *N,N*-Diethyl-4-methoxy-2-methylbenzo[*d*]naphtho[1,2-*f*][1,3,2]dioxaphosphepin-6-amin (0,749 g; 1,96 mmol) in Toluol (10 ml) wurde 1-Naphthol (0,577 g; 3,92 mmol) gegeben. Das Reaktionsgemisch wurde für 69 h bei 110 °C gerührt. Das Lösungsmittel wurde im Vakuum entfernt und der erhaltene Rückstand bei 70 °C / 0,1 mbar getrocknet. Das Rohprodukt wurde durch Säulenchromatografie gereinigt (Dichlormethan, R*_{f}* = 0,94). Ausbeute: 0,540 g (1,194 mmol; 60%).

Elementaranalyse (berechnet für C₂₈H₂₁O₄P = 452,4 g/mol) C 73,93 (74,33); H 5,06 (4,68); P (6,85)%.
³¹P-NMR (CD₂Cl₂): 141,8; 146,4 ppm.
¹H-NMR (CD₂Cl₂): 2,48+2,52 (2s, 3 H); 3,82+4,04 (2s, 3 H); 6,92-8,37 (m, 15 H, Hₐᵣₒₘ) ppm. ¹³C-NMR (CD₂Cl₂): 21,7; 21,8; 56,0; 56,6; 112,8; 113,3; 114,8; 115,0; 115,4; 115,6; 121,2; 121,9; 122,5; 122,6; 124,4; 124,5; 124,6; 125,5; 125,7; 125,9; 126,0; 126,0; 126,4; 126,5;
127,0; 127,1; 127,2; 127,3; 127,8; 128,0; 128,8; 128,8; 129,0; 129,9; 130,4; 132,2; 132,4; 132,6; 134,8; 135,2; 135,3; 135,5; 146,2; 147,1; 148,0; 148,1; 148,2; 152,1; 152,5 ppm.

### 4-(tert-Butyl)-2-methoxy-6-(naphthalen-1-yloxy)benzo[d]naphtho[1,2-f][1,3,2]dioxaphosphepin

### a) Vorstufe, 4-(tert-Butyl)-N,N-diethyl-2-methoxybenzo[d]naphtho[1,2-f][1,3,2]dioxaphosphepin-6-amin

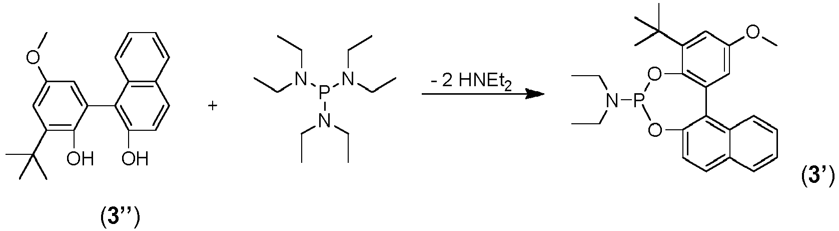

Eine Lösung von 1-(3-(*tert*-Butyl)-2-hydroxy-5-methoxyphenyl)naphthalen-2-ol (0,675 g; 2,09 mmol) in Toluol (10 ml) wurde mit Tris(diethylamino)phosphin (0,517 g; 2,09 mmol) versetzt. Die Reaktionslösung wurde für 62 h bei Raumtemperatur und für 2h bei 70 °C gerührt. Das Lösungsmittel wurde im Vakuum entfernt und der Rückstand getrocknet. Ausbeute: 0,820 g (1,936 mmol; 92 %).

Elementaranalyse (berechnet für C₂₅H₃₀NO₃P = 423,5 g/mol) C 70,98 (70,90); H 7,28 (7,14); N 3,22 (3,31); P 7,36 (7,31)%.
³¹P-NMR (CD₂Cl₂): 149,6 ppm.

### b) 1-Naphthylphosphit

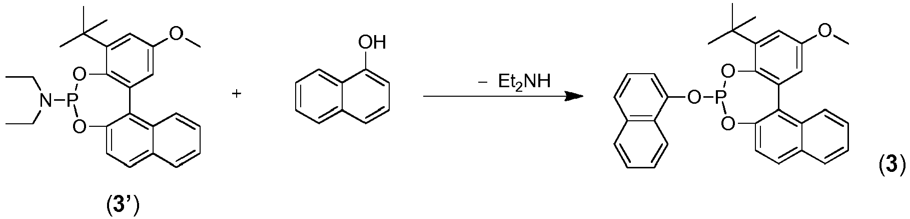

Zu einer gerührten Lösung von 4-(*tert*-Butyl)-*N,N*-diethyl-2-methoxybenzo[*d*]naphtho[1,2-*f*][1,3,2]dioxaphosphepin-6-amin (0,783 g; 1,849 mmol) in Toluol (10 ml) wurde 1-Naphthol (0,533 g; 3,697 mmol) gegeben. Das Reaktionsgemisch wurde für 69 h bei 110 °C gerührt. Das Lösungsmittel wurde im Vakuum entfernt und der erhaltene Rückstand bei 70 °C / 0,1 mbar getrocknet. Das Rohprodukt wurde durch Säulenchromatografie gereinigt (Hexan/Essigsäureethylester, 8:1, R*_{f}* = 0,38). Ausbeute: 0,430 g (0,870 mmol; 47%). Elementaranalyse (berechnet für C₃₁H₂₇O₄P = 494,52 g/mol) C 74,48 (75,29); H 5,71 (5,50); P (6,26)%.
³¹P-NMR (CD₂Cl₂): 144,5 ppm.
¹H-NMR (CD₂Cl₂): 1,59 (s, 9 H); 3,86 (s, 3 H); 7,04 (m, 1 H); 7,16 (m, 1 H,); 7,37-7,51 (m, 3 H); 7,51-7,62 (m, 4 H); 7,68-7,75 (m, 1 H); 7,88-8,00 (m, 3 H); 8,07-8,22 (m, 2 H) ppm.
¹³C-NMR (CD₂Cl₂): 31,3; 31,3; 35,8; 56,0; 115,0; 115,1; 115,2; 115,3; 121,6; 122,4; 124,7; 125,5; 125,9; 125,9; 126,0; 126,6; 127,1; 127,2; 127,7; 128,1; 128,9; 129,9; 130,6; 130,6; 132,3; 132,8; 135,3; 141,9; 144,2; 144,3; 146,6; 146,7; 148,1; 148,2; 156,0 ppm.

### 9-(tert-Butyl)-4-methoxy-2-methyl-6-(naphthalen-1-yloxy)dibenzo[d,f][1,3,2]dioxa-phosphepin

### a) Vorstufe, 9-(tert-Butyl)-N,N-diethyl-4-methoxy-2-methyldibenzo[d,f][1,3,2]dioxaphosphepin-6-amin.

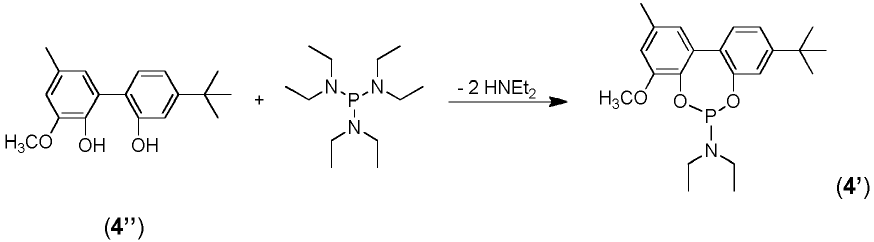

Tris(diethylamino)phosphin (0,592 g; 2,393 mmol) und 4'-(*tert*-Butyl)-3-methoxy-5-methyl-[1,1'-biphenyl]-2,2'-diol (0,685 g; 2,393 mmol), wurden zusammengegeben und dann mit Toluol (9 ml) versetzt. Die klare Lösung wurde über Nacht bei Raumtemperatur gerührt, das Lösungsmittel im Vakuum entfernt und das erhaltene Öl für 2 h bei 70 °C / 0,1 mbar getrocknet. Ausbeute: 0,835 g (2,155 mmol; 90 %).
³¹P-NMR (CD₂Cl₂): 150,3 ppm.
¹H-NMR (CD₂Cl₂): 1,12 (t, 6 H); 1,41 (s, 9 H); 2,43 (s, 3 H); 3,06-3,19 (m, 4 H); 3,91 (s, 3 H); 6,83 (1 H); 6,89 (1 H); 7,23 (1 H); 7,29-7,34 (1 H); 7,41-7,46 (1 H) ppm.

### b) 1-Naphthylphosphit

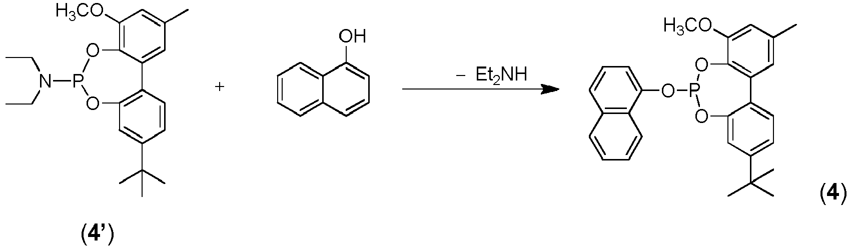

1-Naphthol (0,621 g; 4,309 mmol) und 9-(*tert*-Butyl)-*N,N*-diethyl-4-methoxy-2-methyldibenzo[*d*,*f*][1,3,2]dioxaphosphepin-6-amin (0,835 g; 2,155 mmol), wurden zusammengegeben und dann mit Toluol (12 ml) versetzt. Die klare Lösung wurde für 66 h bei 110 °C gerührt. Dann wurden weitere 0,4 Äquivalente 1-Naphthol zugegeben und für 22 h bei 110 °C gerührt. Das Lösungsmittel wurde im Vakuum entfernt und der ölige Rückstand bei 50 °C / 0,1 mbar getrocknet. Die Substanz wurde durch Säulenchromatografie gereinigt (Dichlormethan, R*_{f}* = 0,70). Ausbeute: 0,710 g (1,549 mmol; 72 %).

Elementaranalyse (berechnet für C₂₈H₂₇O₄P = 458,49 g/ mol) C 73,43 (73,35); H 5,82 (5,94); P 6,72 (6,75) %.

### ³¹P-NMR (CD₂Cl₂): 143,5 ppm.

¹H-NMR (CD₂Cl₂): 1,40 (s, 9 H); 2,47 (3 H); 3,91 (s, 3 H); 6,90 (1 H); 6,97 (1 H); 7,33 (1 H); 7,39-7,44 (1 H); 7,48-7,55 (3 H); 7,57-7,64 (2 H); 7,71-7,76 (1 H); 7,90-7,97 (1 H); 8,29-8,36 (1 H) ppm.
¹³C-NMR (CD₂Cl₂): 21,7; 31,4; 35,0; 56,2; 112,9; 115,3; (d, *J*_{CP}= 13 Hz); 119,4; 121,8; 122,6; 123,0; 124,5; 126,0; 126,5; 127,1; 127,8; 128,0; 128,4; 129,8; 132,1; 135,3; 136,0; 148,2 (d, *J*_{CP}= 7 Hz); 149,3 (d, *J*_{CP}= 6 Hz); 151,9; 153,6 ppm.
ESI-TOF/HRMS: *m*/*e* 459,17232 (M+H)⁺.

### 4-Methoxy-2,10-dimethyl-6-(naphthalen-1-yloxy)dibenzo[d,f][1,3,2]dioxaphosphepin

### a) Vorstufe, N,N-Diethyl-4-methoxy-2,10-dimethyldibenzo[d,f][1,3,2]dioxaphosphepin-6-amin.

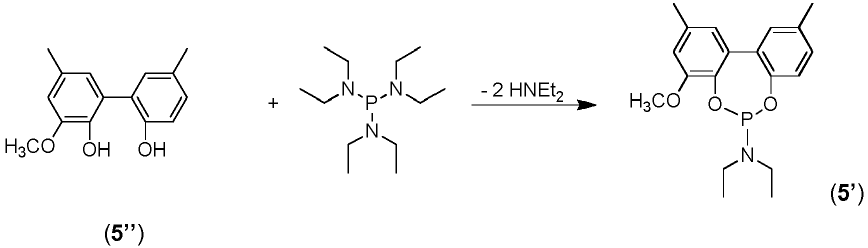

Tris(diethylamino)phosphin (0,578 g; 2,336 mmol) und 3-Methoxy-5,5'-dimethyl-[1,1'-biphenyl]-2,2'-diol (0,571 g; 2,336 mmol), wurden zusammengegeben und dann mit Toluol (9 ml) versetzt. Die klare Lösung wurde über Nacht bei Raumtemperatur gerührt. Das Lösungsmittel wurde im Vakuum entfernt und das zurückgebliebene Öl für 2 h bei 70 °C / 0,1 mbar getrocknet. Ausbeute: 0,733 g (2,123 mmol; 91 %).
³¹P-NMR (CD₂Cl₂): 149,8 ppm.
¹H-NMR (CD₂Cl₂): 1,11 (t, 6 H); 2,43 (6 H); 3,05-3,18 (2m, 4 H); 3,91 (s, 3 H); 6,83 (1 H); 6,90 (1 H,); 7,04-7,10 (1 H); 7,15-7,21 (1 H); 7,31 (1 H) ppm.

### b) 1-Naphthylphosphit

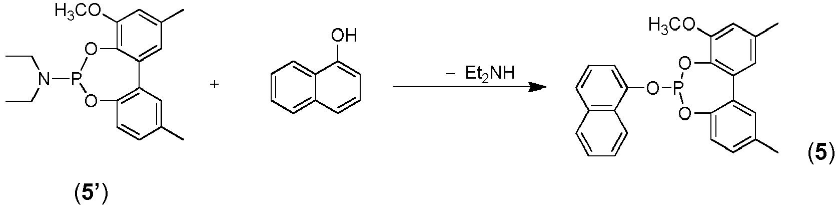

1-Naphthol (0,612 g; 4,245 mmol) und *N,N*-Diethyl-4-methoxy-2,10-dimethyldibenzo[*d*,*f*][1,3,2]dioxa-phosphepin-6-amin (0,733 g; 2,123 mmol), wurden zusammengegeben und dann mit Toluol (12 ml) versetzt. Die klare Lösung wurde für 66 h bei 110 °C gerührt. Das Lösungsmittel wurde im Vakuum entfernt und der ölige Rückstand getrocknet. Die Substanz wurde durch Säulenchromatografie gereinigt (Dichlormethan, R*_{f}* = 0,77). Ausbeute: 0,500 g (1,201 mmol; 57 %).

Elementaranalyse (berechnet für C₂₅H₂₁O₄P = 416,41 g/ mol) C 72,24 (72,11); H 5,11 (5,08); P 7,68 (7,44) %.
³¹P-NMR (CD₂Cl₂): 143,8 ppm.
¹H-NMR (CD₂Cl₂): 2,48 (6 H); 3,90 (s, 3 H); 6,91 (1 H); 7,01 (1 H); 7,21-7,30 (2 H); 7,33 (1 H); 7,48-7,56 (2 H); 7,58-7,65 (2 H); 7,72-7,77 (1 H); 7,91-8,00 (1 H); 8,29-8,37 (1 H) ppm. ¹³C-NMR (CD₂Cl₂): 21,1; 21,7; 56,3; 113,0; 115,3 (d, *J*_{CP}= 13 Hz); 121,9; 122,1; 122,6; 124,6; 126,0; 126,5; 127,0; 127,8; 128,0; 130,3; 130,8; 131,3; 132,2; 135,3; 135,6; 136,0; 136,1; 147,2; 148,2; 151,9 ppm.
ESI-TOF/HRMS: *m*/*e* 417,12522 (M+H)⁺.

### 4-Methoxy-2,8,10-trimethyl-6-(naphthalin-1-yloxy)dibenzo[d,f][1,3,2]dioxa-phosphepin

### a) Vorstufe, N,N-Diethyl-4-methoxy-2,8,10-trimethyldibenzo[d,f][1,3,2]dioxaphosphepin-6-amin

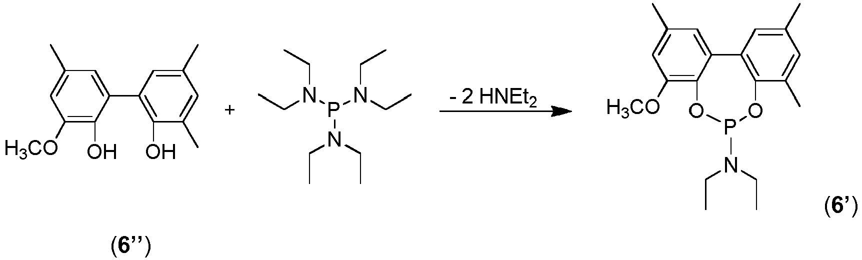

Eine Suspension von 3-Methoxy-3',5,5'-trimethyl-[1,1'-biphenyl]-2,2'-diol (1,304 g; 5,1 mmol) in Toluol (9 ml) wird zunächst 20 min bei Raumtemperatur gerührt und dann tropfenweise mit Tris(diethylamino)phosphin (1,25 g; 5,1 mmol) versetzt. Die Mischung wird kurzzeitig homogen, bevor ein weißer Niederschlag ausfällt. Man rührt 20 h bei Raumtemperatur, filtriert und engt das Filtrat im Vakuum zur Trockne ein und lässt das Rohprodukt mehrere Tage stehen. Die gebildeten Kristalle werden von der Mutterlauge befreit, mit wenig Cyclohexan gewaschen und im Vakuum getrocknet. Ausbeute: 0,497 g (1,38 mmol; 27 %).
Elementaranalyse (berechnet für C₂₀H₂₆O₃NP = 359,4 g/ mol) C 66,90 (66,84); H 7,07 (7,29); N, 4,14 (3,90); P 8,38 (8,62) %.
³¹P-NMR (CD₂Cl₂): 147,6 ppm.
ESI-TOF/HRMS: *m*/*e* 360,17237 (M+H)⁺.

### b) 1-Naphthylphosphit

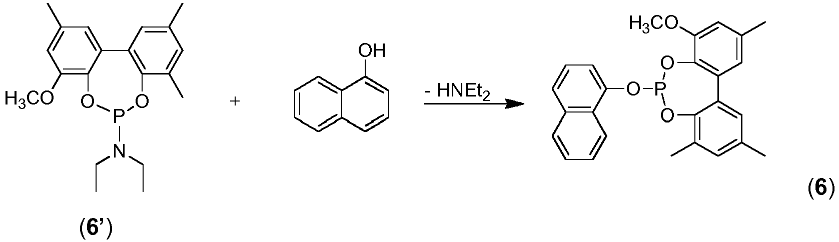

1-Naphthol (0,566 g; 3,92 mmol) und N,N-Diethyl-4-methoxy-2,8,10-trimethyldibenzo[d,f][1,3,2]dioxaphosphepin-6-amin (0,706 g; 1,96 mmol), wurden zusammengegeben und dann mit Toluol (10 ml) versetzt. Die klare Lösung wurde für 31 h bei 110 °C gerührt. Das Lösungsmittel wurde im Vakuum entfernt und der ölige Rückstand getrocknet. Die Substanz wurde durch Säulenchromatografie gereinigt (Heptan/Essigsäureethylester, 85:15, R*_{f}* = 0,39). Ausbeute: 0,359 g (0,83 mmol; 42 %). Elementaranalyse (berechnet für C₂₆H₂₃O₄P = 430,43 g/ mol) C 72,62 (72,55); H 5,55 (5,39); P 6,80 (7,20) %.
³¹P-NMR (CD₂Cl₂): 142,2 ppm.
¹H-NMR (CD₂Cl₂): 2,49 (3 H); 2,51 (3 H); 2,55 (3 H); 3,90 (s, 3 H); 6,91 (1 H); 7,04 (1 H); 7,22 (1 H); 7,30 (1 H), 7,53-7,69 (m, 4 H); 7,78 (1 H); 7,94-8,01 (m, 1 H); 8,41 (m, 1 H) ppm.
EI-MS: *m*/*e* 430 (4%, M+H⁺).

### 8-Methoxy-2,3,10-trimethyl-6-(naphthalen-1-yloxy)dibenzo[d,f][1,3,2]dioxaphos-phepin

### a) Vorstufe, N,N-Diethyl-8-methoxy-2,3,10-trimethyldibenzo[d,f][1,3,2]dioxaphosphepin-6-amin

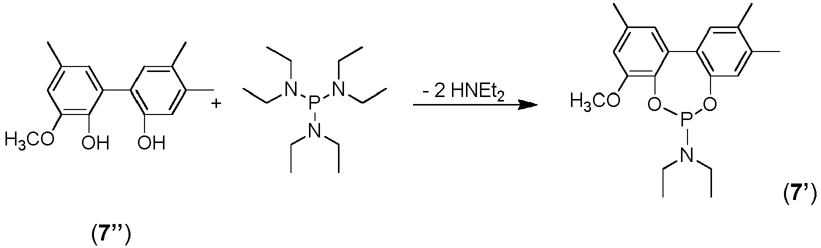

Eine Suspension von 3-Methoxy-4',5,5'-trimethyl-[1,1'-biphenyl]-2,2'-diol (1,499 g; 5,8 mmol) in Toluol (9 ml) wird zunächst 20 min bei Raumtemperatur gerührt und dann tropfenweise mit Tris(diethylamino)phosphin (1,435 g; 5,8 mmol) versetzt. Die Mischung wird kurzzeitig homogen, bevor ein weißer Niederschlag ausfällt. Man rührt 20 h bei Raumtemperatur, filtriert, engt das Filtrat im Vakuum zur Trockne ein und reinigt den Rückstand mittels Säulenchromatografie (Heptan/Ethylacetat, 3:1, R*_{f}*= 0,65). Ausbeute: 0,791 g (2,2 mmol; 38%). Elementaranalyse (berechnet für C₂₀H₂₆O₃NP = 359,20 g/ mol) C 66,84 (66,84); H 7,38 (7,29); N 3,82 (3,90); P 8,66 (8,62) %.
³¹P-NMR (CD₂Cl₂): 149,4 ppm.
ESI-TOF/HRMS: *m*/*e* 360,17237 (M+H)⁺.

### b) 1-Naphthylphosphit

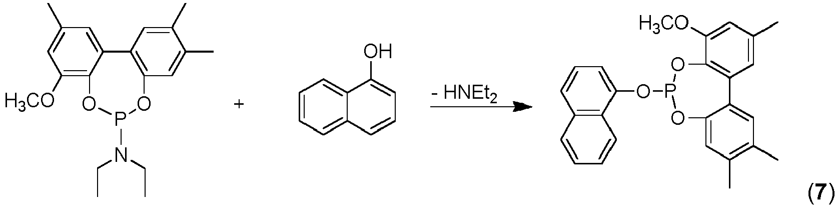

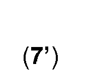

1-Naphthol (0,952 g; 6,6 mmol) und N,N-Diethyl-8-methoxy-2,3,10-trimethyl-dibenzo[d,f][1,3,2]dioxaphosphepin-6-amin (0,706 g; 3,32 mmol), wurden zusammengegeben und mit Toluol (10 ml) versetzt. Die Lösung wird für 31 h bei 110 °C gerührt. Das Lösungsmittel wurde im Vakuum entfernt und der ölige Rückstand getrocknet. Die Substanz wurde durch Säulenchromatografie gereinigt (Heptan/Essigsäureethylester, 4:1, R_{f} = 0,51). Ausbeute: 0,887 g (2,06 mmol; 63 %).

Elementaranalyse (berechnet für C₂₆H₂₃O₄P = 430,43 g/ mol) C 72,58 (72,55); H 5,44 (5,39); P 6,80 (7,20) %.
³¹P-NMR (CD₂Cl₂): 142,9 ppm.
¹H-NMR (CD₂Cl₂): 2,32 (3 H); 2,35 (3 H); 2,45 (3 H); 3,90 (s, 3 H); 6,86 (1 H); 6,94 (1 H); 7,04 (1 H); 7,32 (1 H), 7,43-7,50 (m, 2 H); 7,54-7,61 m, 2 H); 7,67-7,74 (m, 1 H); 7,91 (m, 1 H); 8,27 (m, 1 H) ppm.
EI-MS: *m*/*e* 430 (6%, M+H⁺)

### Arbeitsvorschrift für die Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl getrocknet und unter Argon destilliert. Als Olefin wurde ein Octengemisch eingesetzt: n-Octene (Oxeno GmbH, Octenisomerengemisch aus 1-Octen: ∼3 %; *cis*+*trans*-2-Octen: ∼49%; *cis*+*trans*-3-Octen: ∼29%; *cis*+*trans*-Octen-4: ∼16%; gerüstisomere Octene: ∼3 % wurden mehrere Stunden über Natrium am Rückfluß erhitzt und unter Argon destilliert.

Für die Versuche wurden im Autoklaven unter Argonatmosphäre folgende Lösungen des Rhodiums in Form von [(acac)Rh(COD)] (acac = Acetylacetonat-Anion; COD = 1,5-Cyclooctadien, Umicore) als Katalysatorvorstufe in Toluol eingefüllt: Für Versuche mit 100 ppm-m Rhodium 10 ml einer 4,31 millimolaren, für 40 bzw. 60 ppm-m die gleiche Menge einer entsprechend verdünnten Lösung. Anschließend wurde die entsprechende Menge der in Toluol gelösten Phosphitverbindung (5 Ligandäquivalente pro Rhodium) zugemischt. Durch Zugabe von weiterem Toluol (Die Gesamtmasse an Toluol wurde bestimmt für die GC-Analyse, s.u.) wurde das Anfangsvolumen der Katalysatorlösung auf 41,0 ml eingestellt. Die jeweils eingebrachte Masse an Toluol wurde bestimmt. Einwaage n-Octene: 10,70 g (95,35 mmol). Der Autoklav wurde bei einem Gesamtgasdruck (Synthesegas: Linde; H₂ (99,999%): CO (99,997%) = 1:1) von a) 42 bar für einen Enddruck von 50 bar bzw. b) 12 bar für den Enddruck von 20 bar unter Rühren (1500 U/min) auf die jeweils angegebenen Temperaturen aufgeheizt. Nach Erreichen der Reaktionstemperatur wurde der Synthesegasdruck auf a) 48,5 bar für einen Enddruck von 50 bar bzw. b) 19,5 bar für einen Enddruck von 20 bar erhöht und das Edukt mit einem in der Druckpipette eingestellten Überdruck von ca. 3 bar zugepresst. Die Reaktion wurde bei konstantem Druck von jeweils 50 bzw. 20 bar (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm, Die quantitative Bestimmung von Restolefin und Aldehyd erfolgte gegen das Lösungsmittel Toluol als internen Standard.

### Katalyseergebnisse

Die Ergebnisse der Katalyseversuche sind in der Tabellen 1 zusammengefasst.

**Tabelle 1:**

| **Ligand** | **p (bar)** | **T (°C)** | **t (h)** | **[Rh] (ppm)** | **L/Rh** | **Ausb. (%)** | **n-Sel. (%)** |
|---|---|---|---|---|---|---|---|
| **1** | 20 | 120 | 4 | 100 | 5 | 90 | 24,9 |
| **2*** | 20 | 120 | 4 | 100 | 5 | 89 | 27,7 |
| **3*** | 20 | 120 | 4 | 100 | 5 | 90 | 33,5 |
| **4*** | 20 | 120 | 4 | 100 | 5 | 95 | 26,1 |
| **5*** | 20 | 120 | 4 | 100 | 5 | 92 | 25,7 |
| **6*** | 20 | 120 | 4 | 100 | 5 | 91 | 30,8 |
| **7*** | 20 | 120 | 4 | 100 | 5 | 94 | 25,8 |

| | | | | | | | |
|---|---|---|---|---|---|---|---|
| * Monophosphite, welche aus erfindungsgemäße Verbindung hergestellt wurden Olefin: n-Octene (Oxeno GmbH) Solvens: Toluol Verhältnis Ligand/Rhodium (L/Rh): 5 : 1 | | | | | | | |

Alle Liganden, welche aus erfindungsgemäßen Verbindungen hergestellt wurden, weißen eine höhere Selektivität auf, als der Vergleichsligand (**1**). Teilweise sogar in Kombination mit einer gesteigerten Ausbeute, was besonders vorteilhaft ist.

Anhand der oben beschriebenen Versuche konnte gezeigt werden, dass die gestellte Aufgabe durch die erfindungsgemäßen Verbindungen gelöst wird.

## Patentansprüche

1. Verbindung, welche eine der allgemeinen Strukturen (I) oder **(II)** aufweist: 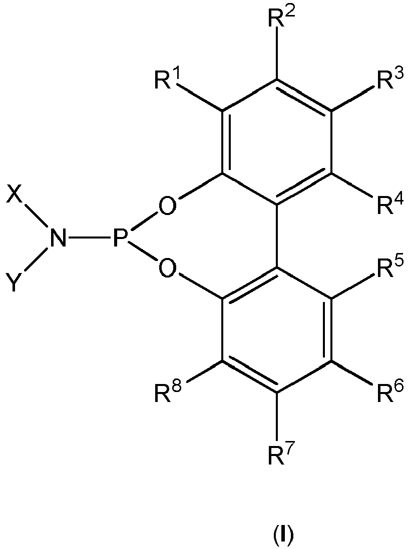 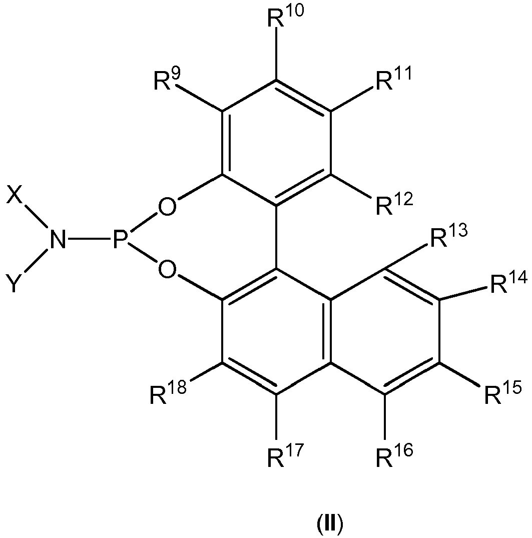 wobei R¹, R², R³, R⁴, R⁵, R⁶, R⁷, R⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R⁹, R¹⁰, R¹¹, R¹², R¹³, R¹⁴, R¹⁵ R¹⁶ R¹⁷, R¹⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
wobei die genannten Alkyl- und Arylgruppen wie folgt substituiert sein können:
(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl;
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl,-COOH, -OH, -SO₃H; -SO₃Na, -NO₂, -CN, -NH₂, -N[(C₁-C₁₂)-Alkyl]₂;
und X und Y ausgewählt sind aus:
- (C₁-C₁₂)-Alkyl, -(C₃-C₁₂)-Cycloalkyl, -(C₃-C₁₂)-Heterocycloalkyl, -(C₆-C₂₀)-Aryl,
wobei die genannten Alkyl- und Arylgruppen wie folgt substituiert sein können: (C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl;
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl,-COOH, -OH, -SO₃H; -SO₃Na, -NO₂, -CN, -NH₂, -N[(C₁-C₁₂)-Alkyl]₂;
und die beiden Reste mindestens eines der vier folgenden Restepaare nicht für den gleichen Rest stehen: R¹ und R⁸, R² und R⁷, R³ und R⁶, R⁴ und R⁵.

2. Verbindung nach Anspruch 1,
wobei X und Y ausgewählt sind aus:
-(C₁-C₁₂)-Alkyl, -(C₃-C₁₂)-Cycloalkyl, -(C₆-C₂₀)-Aryl.

3. Verbindung nach einem der Ansprüche 1 oder 2,
wobei X und Y ausgewählt sind aus:
-(C₁-C₁₂)-Alkyl, -(C₃-C₁₂)-Cycloalkyl.

4. Verbindung nach einem der Ansprüche 1 bis 3,
wobei R¹, R², R³, R⁴, R⁵, R⁶, R⁷, R⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen.

5. Verbindung nach einem der Ansprüche 1 bis 4,
wobei R⁹, R¹⁰, R¹¹, R¹², R¹³, R¹⁴, R¹⁵, R¹⁶ R¹⁷, R¹⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen.

6. Verbindung nach einem der Ansprüche 1 bis 5,
wobei R¹, R², R³, R⁴, R⁵, R⁶, R⁷, R⁸ ausgewählt sind aus:
-H, -(C₁-C₁₂)-Alkyl, -O-(C₁-C₁₂)-Alkyl, -O-(C₆-C₂₀)-Aryl.

7. Verbindung nach einem der Ansprüche 1 bis 6,
wobei R⁹, R¹⁰, R¹¹, R¹², R¹³, R¹⁴, R¹⁵, R¹⁶ R¹⁷, R¹⁸ ausgewählt sind aus:
-H, -(C₁-C₁₂)-Alkyl, -O-(C₁-C₁₂)-Alkyl, -O-(C₆-C₂₀)-Aryl.

8. Verbindung nach einem der Ansprüche 1 bis 7,
wobei R¹, R², R³, R⁴, R⁵, R⁶, R⁷, R⁸ ausgewählt sind aus:
-H, -(C₁-C₁₂)-Alkyl, -O-(C₁-C₁₂)-Alkyl.

9. Verbindung nach einem der Ansprüche 1 bis 8,
wobei R⁹, R¹⁰, R¹¹, R¹², R¹³, R¹⁴, R¹⁵ R¹⁶ R¹⁷, R¹⁸ ausgewählt sind aus:
-H, -(C₁-C₁₂)-Alkyl, -O-(C₁-C₁₂)-Alkyl.

10. Verbindung nach einem der Ansprüche 1 bis 9,
welche eine der folgenden Strukturen (**2'**) bis (**7'**) aufweist:

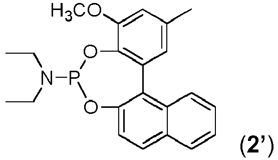

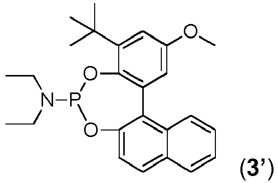

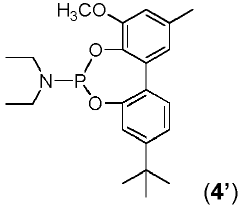

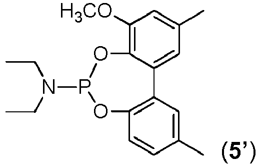

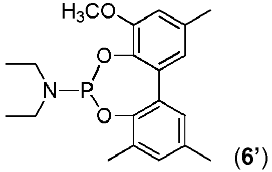

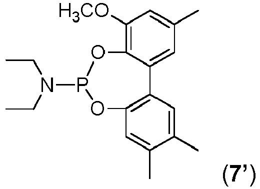

11. Verbindung nach einem der Ansprüche 1 bis 10,
wobei die Verbindung die allgemeinen Strukturen (**I**) aufweist.

12. Verbindung nach einem der Ansprüche 1 bis 10,
wobei die Verbindung die allgemeinen Strukturen (**II**) aufweist.

13. Verwendung einer Verbindung nach einem der Ansprüche 1 bis 12,
zur Herstellung von Monophosphiten.

14. Verfahren umfassend die Verfahrensschritte:
a) Vorlegen einer Verbindung, welche eine der allgemeinen Strukturen (**III**) oder (**IV**) aufweist: 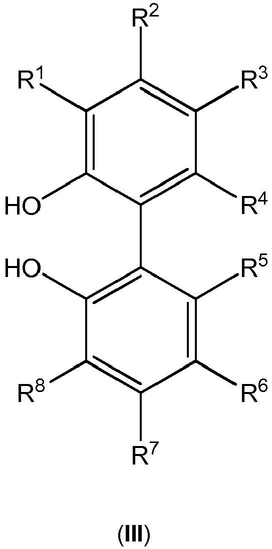 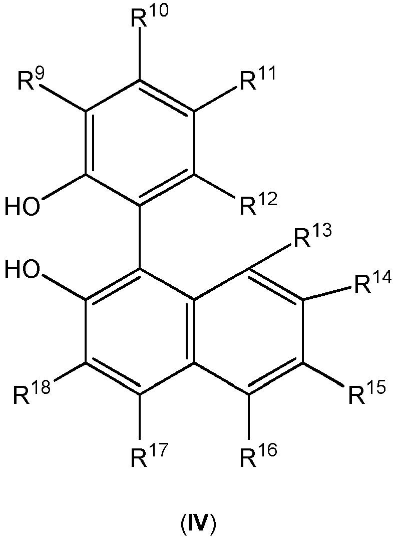 wobei
R¹, R², R³, R⁴, R⁵, R⁶, R⁷, R⁸ ausgewählt sind aus:
- H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R⁹, R¹⁰, R¹¹, R¹², R¹³, R¹⁴, R¹⁵, R¹⁶ R¹⁷, R¹⁸ ausgewählt sind aus:
- H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
wobei die genannten Alkyl- und Arylgruppen wie folgt substituiert sein können:
(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl;
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl,-COOH, -OH, -SO₃H; -SO₃Na, -NO₂, -CN, -NH₂, -N[(C₁-C₁₂)-Alkyl]₂;
und die beiden Reste mindestens eines der vier folgenden Restepaare nicht für den gleichen Rest stehen: R¹ und R⁸, R² und R⁷, R³ und R⁶, R⁴ und R⁵;
b) Hinzugeben einer Verbindung, welche die allgemeinen Struktur **(V)** aufweist: 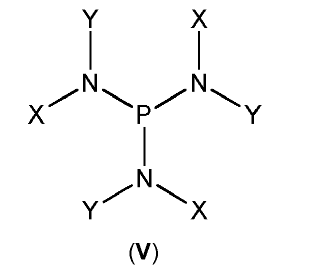 wobei X und Y ausgewählt sind aus:
-(C₁-C₁₂)-Alkyl, -(C₃-C₁₂)-Cycloalkyl, -(C₃-C₁₂)-Heterocycloalkyl, -(C₆-C₂₀)-Aryl;
c) Durchmischen der Verbindungen aus Verfahrensschritt a) und b) wobei **(V)** mit (**III**) zu **(I)** umgesetzt wird, beziehungsweise **(V)** mit (**IV**) zu (**II**) umgesetzt wird: 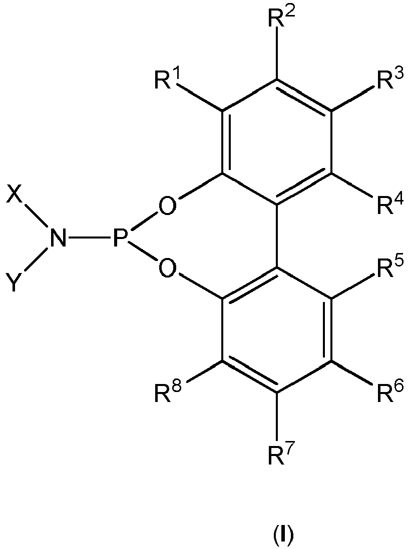 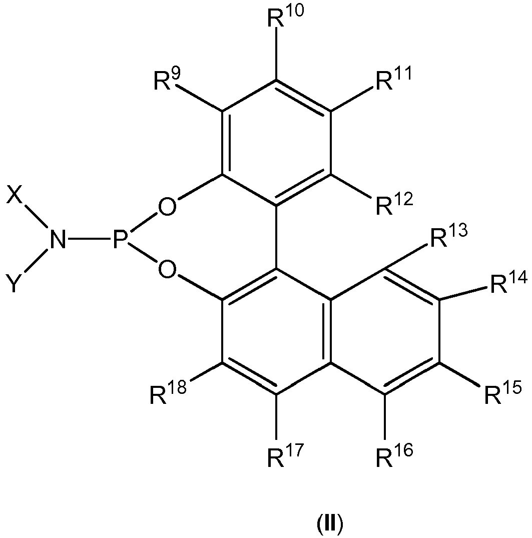 wobei
R¹, R², R³, R⁴, R⁵, R⁶, R⁷, R⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R⁹, R¹⁰, R¹¹, R¹², R¹³, R¹⁴, R¹⁵ R¹⁶ R¹⁷, R¹⁸ ausgewählt sind aus:
- H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
wobei die genannten Alkyl- und Arylgruppen wie folgt substituiert sein können:
(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl;
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, - COOH, -OH, -SO₃H; -SO₃Na, -NO₂, -CN, -NH₂, -N[(C₁-C₁₂)-Alkyl]₂;
und X und Y ausgewählt sind aus:
- (C₁-C₁₂)-Alkyl, -(C₃-C₁₂)-Cycloalkyl, -(C₃-C₁₂)-Heterocycloalkyl, -(C₆-C₂₀)-Aryl,
wobei die genannten Alkyl- und Arylgruppen wie folgt substituiert sein können:
(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl;
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, - COOH, -OH, -SO₃H; -SO₃Na, -NO₂, -CN, -NH₂, -N[(C₁-C₁₂)-Alkyl]₂;
und die beiden Reste mindestens eines der vier folgenden Restepaare nicht für den gleichen Rest stehen: R¹ und R⁸, R² und R⁷, R³ und R⁶, R⁴ und R⁵.

15. Verfahren nach Anspruch 14,
umfassend den zusätzlichen Verfahrensschritt:
d) Zugabe von Naphthol zu dem Reaktionsprodukt aus Verfahrensschritt c).

## Claims

1. Compound which has one of the general structures (I) or (II) : 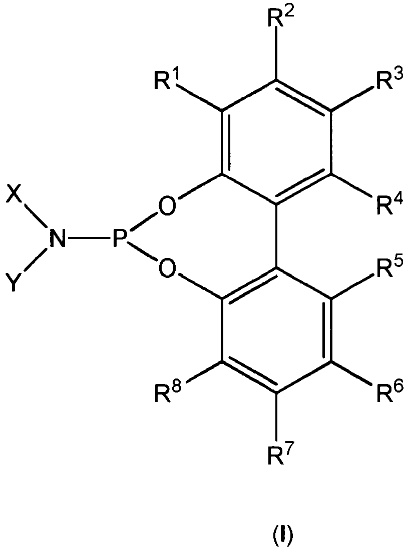 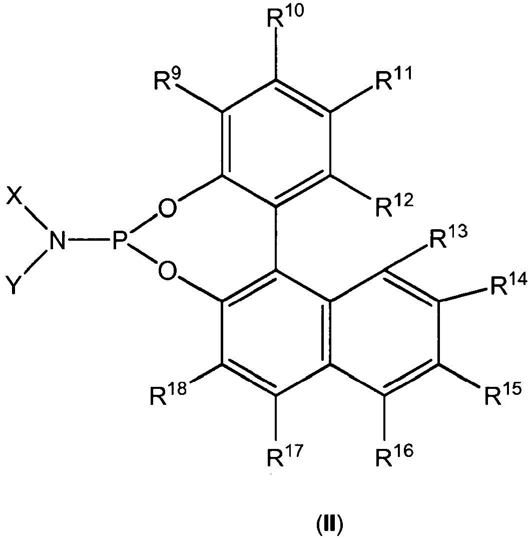 where
R¹, R², R³, R⁴, R⁵, R⁶, R⁷, R⁸ are selected from among:
- H, (C₁-C₁₂)-alkyl, -0- (C₁-C₁₂) -alkyl, -O-(C₆-C₂₀)-aryl, -(C₆-C₂₀)-aryl, -S-alkyl, -S-aryl, halogen, -COO- (C₁-C₁₂) -alkyl, -CONH-(C₁-C₁₂)-alkyl, -CO-(C₁-C₁₂)-alkyl, -CO-(C₆-C₂₀)-aryl, -COOH, -OH, -SO₃H, -CN, -NH₂, -N[(C₁-C₁₂)-alkyl]₂;
R⁹, R¹⁰, R¹¹, R¹², R¹³, R¹⁴, R¹⁵, R¹⁶, R¹⁷, R¹⁸ are selected from among:
- H, (C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen, -COO-(C₁-C₁₂)-alkyl, -CONH-(C₁-C₁₂)-alkyl, -CO-(C₁-C₁₂)-alkyl, -CO-(C₆-C₂₀)-aryl, -COOH, -OH, -SO₃H, -NH₂, -N[(C₁-C₁₂)-alkyl]₂;
where the alkyl and aryl groups mentioned can be substituted as follows: (C₁-C₁₂)-alkyl and O-(C₁-C₁₂)-alkyl can each be unsubstituted or substituted by one or more identical or different radicals selected from among (C₃-C₁₂)-cycloalkyl, (C₃-C₁₂)-heterocycloalkyl, (C₆-C₂₀)-aryl, fluorine, chlorine, cyano, formyl, acyl and alkoxycarbonyl; substituted -(C₆-C₂₀)-aryl groups and -(C₆-C₂₀)-aryl-(C₆-C₂₀)-aryl groups can, depending on the ring size, have one or more substituents; the substituents are selected independently from among -H, (C₁-C₁₂)-alkyl, -O- (C₁-C₁₂) -alkyl, -O-(C₆-C₂₀) - aryl, - (C₆-C₂₀) -aryl, -halogen, -COO-(C₁-C₁₂)-alkyl, -CONH- (C₁-C₁₂) -alkyl, - (C₆-C₂₀) -aryl-CON[(C₁-C₁₂) - alkyl]₂, -CO-(C₁-C₁₂)-alkyl, -CO-(C₆-C₂₀) -aryl, -COOH, -OH, -SO₃H, -SO₃Na, -NO₂, -CN, -NH₂, -N[(C₁-C₁₂)-alkyl]₂;
and X and Y are selected from among:
- C₁-C₁₂)-alkyl, -(C₃-C₁₂)-cycloalkyl, -(C₃-C₁₂)-heterocycloalkyl, -(C₆-C₂₀)-aryl,
where the alkyl and aryl groups mentioned can be substituted as follows: (C₁-C₁₂) -alkyl and O-(C₁-C₁₂)-alkyl can each be unsubstituted or substituted by one or more identical or different radicals selected from among (C₃-C₁₂) -cycloalkyl, (C₃-C₁₂)-heterocycloalkyl, (C₆-C₂₀)-aryl, fluorine, chlorine, cyano, formyl, acyl and alkoxycarbonyl; substituted -(C₆-C₂₀)-aryl groups and -(C₆-C₂₀)-aryl-(C₆-C₂₀)-aryl groups can, depending on the ring size, have one or more substituents; the substituents are selected independently from among -H, (C₁-C₁₂)-alkyl, -O-(C₁-C₁₂)-alkyl, -O-(C₆-C₂₀)-aryl, - (C₆-C₂₀) -aryl, -halogen, -COO-(C₁-C₁₂)-alkyl, -CONH- (C₁-C₁₂) -alkyl, - (C₆-C₂₀) -aryl-CON[(C₁-C₁₂)-alkyl] 2, -CO-(C₁-C₁₂)-alkyl, -CO-(C₆-C₂₀) -aryl, -COOH, -OH, -SO₃H, -SO₃Na, -NO₂, -CN, -NH₂, -N[(C₁-C₁₂)-alkyl]₂;
and the two radicals of at least one of the following four pairs of radicals are not the same radical: R¹ and R⁸, R² and R⁷, R³ and R⁶, R⁴ and R⁵.

2. Compound according to Claim 1,
wherein X and Y are selected from among:
- C₁-C₁₂)-alkyl, -(C₃-C₁₂)-cycloalkyl, -(C₆-C₂₀)-aryl.

3. Compound according to either Claim 1 or 2,
wherein X and Y are selected from among:
-(C₁-C₁₂)-alkyl, -(C₃-C₁₂)-cycloalkyl.

4. Compound according to any of Claims 1 to 3,
wherein R¹, R², R³, R⁴, R⁵, R⁶, R⁷, R⁸ are selected from among:
-H, (C₁-C₁₂)-alkyl, -0- (C₁-C₁₂) -alkyl, -O-(C₆-C₂₀)-aryl, -(C₆-C₂₀)-aryl, -S-alkyl, -S-aryl, halogen.

5. Compound according to any of Claims 1 to 4,
wherein R⁹, R¹⁰, R¹¹, R¹², R¹³, R¹⁴, R¹⁵, R¹⁶, R¹⁷, R¹⁸ are selected from among:
-H, (C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen.

6. Compound according to any of Claims 1 to 5,
wherein R¹, R², R³, R⁴, R⁵, R⁶, R⁷, R⁸ are selected from among:
-H, (C₁-C₁₂)-alkyl, -O-(C₁-C₁₂)-alkyl, -O-(C₆-C₂₀)-aryl.

7. Compound according to any of Claims 1 to 6,
wherein R⁹, R¹⁰, R¹¹, R¹², R¹³, R¹⁴, R¹⁵, R¹⁶, R¹⁷, R¹⁸ are selected from among:
-H, (C₁-C₁₂)-alkyl, -O-(C₁-C₁₂)-alkyl, -O-(C₆-C₂₀)-aryl.

8. Compound according to any of Claims 1 to 7,
wherein R¹, R², R³, R⁴, R⁵, R⁶, R⁷, R⁸ are selected from among:
-H, (C₁-C₁₂)-alkyl, -O- (C₁-C₁₂) -alkyl.

9. Compound according to any of Claims 1 to 8,
wherein R⁹, R¹⁰, R¹¹, R¹², R¹³, R¹⁴, R¹⁵, R¹⁶, R¹⁷, R¹⁸ are selected from among:
-H, (C₁-C₁₂)-alkyl, -0- (C₁-C₁₂) -alkyl.

10. Compound according to any of Claims 1 to 9, which has one of the structures **(2')** to **(7'):**

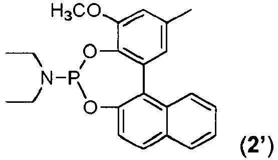

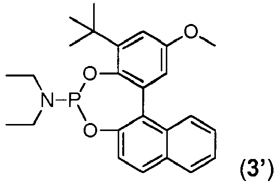

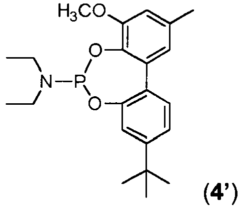

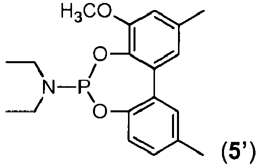

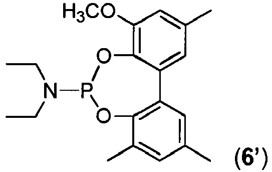

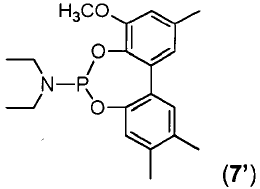

11. Compound according to any of Claims 1 to 10, wherein the compound has the general structures **(I).**

12. Compound according to any of Claims 1 to 10,
wherein the compound has the general structures **(II).**

13. Use of a compound according to any of Claims 1 to 12 for preparing monophosphites.

14. Process comprising the process steps:
a) initial charging of a compound having one of the general structures **(III)** or **(IV):** 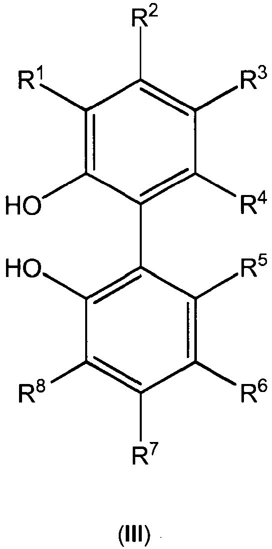 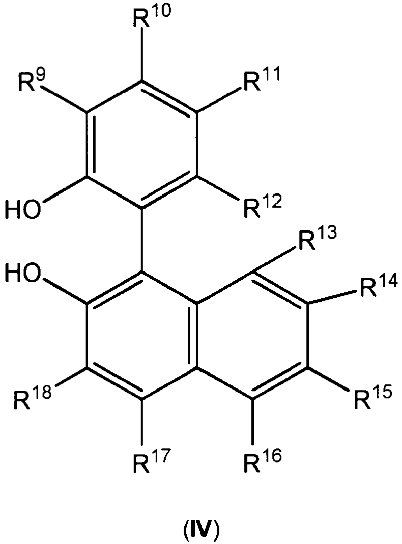
where R¹, R², R³, R⁴, R⁵, R⁶, R⁷, R⁸ are selected from among:
- H, - (C₁-C₁₂) -alkyl, -O- (C₁-C₁₂) -alkyl, -O-(C₆-C₂₀)-aryl, -(C₆-C₂₀)-aryl, -S-alkyl, -S-aryl, halogen, -COO- (C₁-C₁₂) -alkyl, -CONH-(C₁-C₁₂)-alkyl, -CO-(C₁-C₁₂)-alkyl, -CO-(C₆-C₂₀)-aryl, -COOH, -OH, -SO₃H, -CN, -NH₂, -N[(C₁-C₁₂)-alkyl]₂;
R⁹, R¹⁰, R¹¹, R¹², R¹³, R¹⁴, R¹⁵, R¹⁶, R¹⁷, R¹⁸ are selected from among:
- H, (C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen, -COO-(C₁-C₁₂)-alkyl, -CONH-(C₁-C₁₂)-alkyl, -CO-(C₁-C₁₂)-alkyl, -CO-(C₆-C₂₀)-aryl, -COOH, -OH, -SO₃H, -NH₂, -N[(C₁-C₁₂)-alkyl]₂;
where the alkyl and aryl groups mentioned can be substituted as follows: (C₁-C₁₂) -alkyl and O-(C₁-C₁₂)-alkyl can each be unsubstituted or substituted by one or more identical or different radicals selected from among (C₃-C₁₂) -cycloalkyl, (C₃-C₁₂) - heterocycloalkyl, (C₆-C₂₀)-aryl, fluorine, chlorine, cyano, formyl, acyl and alkoxycarbonyl; substituted -(C₆-C₂₀)-aryl groups and -(C₆-C₂₀)-aryl-(C₆-C₂₀)-aryl groups can, depending on the ring size, have one or more substituents; the substituents are selected independently from among -H, (C₁-C₁₂)-alkyl, -O- (C₁-C₁₂) -alkyl, -O-(C₆-C₂₀)-aryl, - (C₆-C₂₀) -aryl, -halogen, -COO-(C₁-C₁₂)-alkyl, -CONH- (C₁-C₁₂) -alkyl, - (C₆-C₂₀) -aryl-CON[(C₁-C₁₂) - alkyl]₂, -CO-(C₁-C₁₂)-alkyl, -CO- (C₆-C₂₀) -aryl, -COOH, -OH, -SO₃H, -SO₃Na, -NO₂, -CN, -NH₂, -N[(C₁-C₁₂)-alkyl]₂;
and the two radicals of at least one of the following four pairs of radicals are not the same radical: R¹ and R⁸, R² and R⁷, R³ and R⁶, R⁴ and R⁵;
b) addition of a compound having the general structure **(V):** 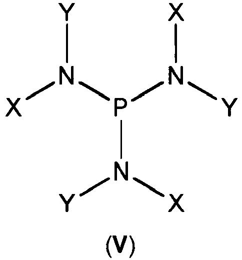 where X and Y are selected from among:
- C₁-C₁₂)-alkyl, - (C₃-C₁₂) -cycloalkyl, - (C₃-C₁₂) - heterocycloalkyl, -(C₆-C₂₀)-aryl;
c) mixing of the compounds from process step a) and b), with **(V)** being reacted with **(III)** to form (**I**) or **(V)** being reacted with **(IV)** to form **(II):** 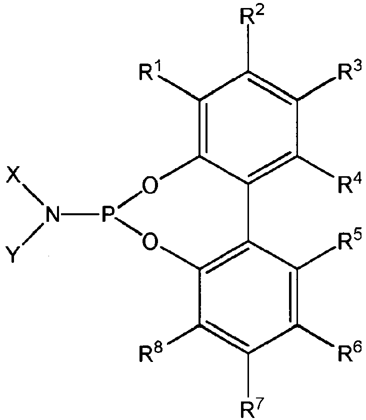 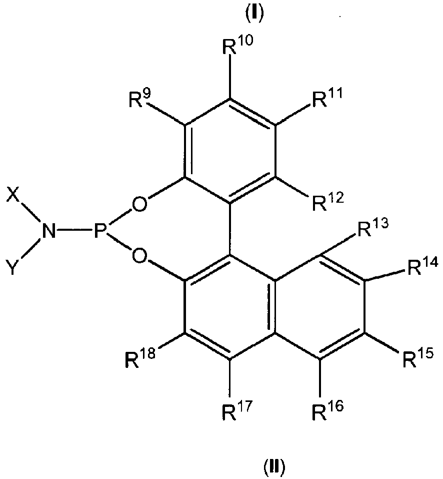 where
R¹, R², R³, R⁴, R⁵, R⁶, R⁷, R⁸ are selected from among:
- H, (C₁-C₁₂)-alkyl, -O- (C₁-C₁₂) -alkyl, -O-(C₆-C₂₀)-aryl, -(C₆-C₂₀)-aryl, -S-alkyl, -S-aryl, halogen, -COO- (C₁-C₁₂) -alkyl, -CONH-(C₁-C₁₂)-alkyl, -CO-(C₁-C₁₂)-alkyl, -CO-(C₆-C₂₀)-aryl, -COOH, -OH, -SO₃H, -CN, -NH₂, -N[(C₁-C₁₂)-alkyl]₂;
R⁹, R¹⁰, R¹¹, R¹², R¹³, R¹⁴, R¹⁵, R¹⁶, R¹⁷, R¹⁸ are selected from among:
-H, (C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen, -COO-(C₁-C₁₂)-alkyl, -CONH-(C₁-C₁₂)-alkyl, -CO-(C₁-C₁₂)-alkyl, -CO-(C₆-C₂₀)-aryl, -COOH, -OH, -SO₃H, -NH₂, -N[(C₁-C₁₂)-alkyl]₂;
where the alkyl and aryl groups mentioned can be substituted as follows:
C₁-C₁₂)-alkyl and O-(C₁-C₁₂)-alkyl can each be unsubstituted or substituted by one or more identical or different radicals selected from among (C₃-C₁₂) -cycloalkyl, (C₃-C₁₂) -heterocycloalkyl, (C₆-C₂₀)-aryl, fluorine, chlorine, cyano, formyl, acyl and alkoxycarbonyl;
substituted -(C₆-C₂₀)-aryl groups and -(C₆-C₂₀)-aryl-(C₆-C₂₀)-aryl groups can, depending on the ring size, have one or more substituents; the substituents are selected independently from among -H, (C₁-C₁₂)-alkyl, -O-(C₁-C₁₂)-alkyl, -O-(C₆-C₂₀)-aryl, -(C₆-C₂₀)-aryl, -halogen, -COO-(C₁-C₁₂)-alkyl, -CONH- (C₁-C₁₂) -alkyl, - (C₆-C₂₀) -aryl-CON[(C₁-C₁₂)-alkyl] 2, -CO-(C₁-C₁₂)-alkyl, -CO-(C₆-C₂₀) -aryl, -COOH, -OH, -SO₃H, -SO₃Na, -NO₂, -CN, -NH₂, -N[(C₁-C₁₂)-alkyl]₂;
and X and Y are selected from among:
- C₁-C₁₂)-alkyl, -(C₃-C₁₂)-cycloalkyl, (C₃-C₁₂)-heterocycloalkyl, -(C₆-C₂₀)-aryl,
where the alkyl and aryl groups mentioned can be substituted as follows:
(C₁-C₁₂) -alkyl and O-(C₁-C₁₂)-alkyl can each be unsubstituted or substituted by one or more identical or different radicals selected from among (C₃-C₁₂)-cycloalkyl, (C₃-C₁₂)-heterocycloalkyl, (C₆-C₂₀)-aryl, fluorine, chlorine, cyano, formyl, acyl and alkoxycarbonyl;
substituted -(C₆-C₂₀)-aryl groups and -(C₆-C₂₀)-aryl-(C₆-C₂₀)-aryl groups can, depending on the ring size, have one or more substituents; the substituents are selected independently from among -H, (C₁-C₁₂)-alkyl, -O-(C₁-C₁₂)-alkyl, -O-(C₆-C₂₀)-aryl, -(C₆-C₂₀)-aryl, -halogen, -COO-(C₁-C₁₂)-alkyl, -CONH- (C₁-C₁₂) -alkyl, - (C₆-C₂₀) -aryl-CON[(C₁-C₁₂)-alkyl] 2, -CO-(C₁-C₁₂)-alkyl, -CO-(C₆-C₂₀)-aryl, -COOH, -OH, -SO₃H, -SO₃Na, -NO₂, -CN, -NH₂, -N[(C₁-C₁₂)-alkyl]₂;
and the two radicals of at least one of the following four pairs of radicals are not the same radical: R¹ and R⁸, R² and R⁷, R³ and R⁶, R⁴ and R⁵.

15. Process according to Claim 14 comprising the additional process step:
d) addition of naphthol to the reaction product from process step c).

## Revendications

1. Composé qui présente une des deux structures générales (I) ou (II) : 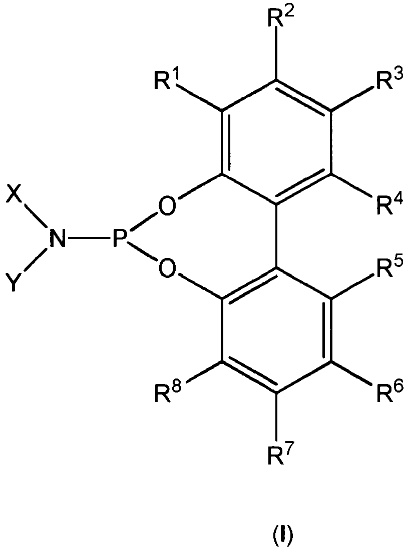 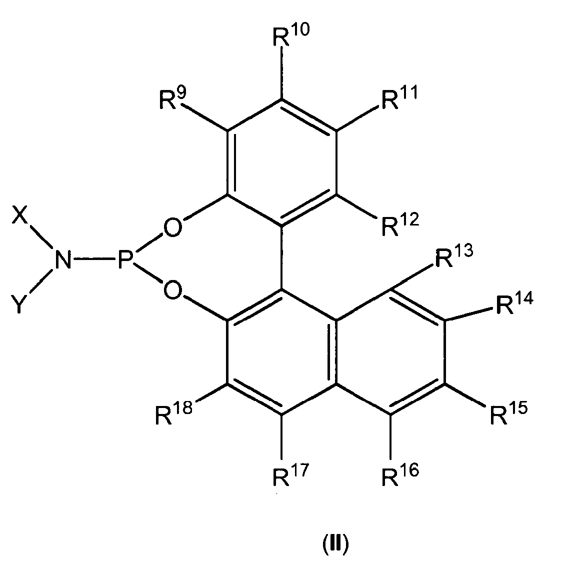 dans lesquelles
R¹, R², R³, R⁴, R⁵, R⁶, R⁷, R⁸ sont choisis parmi : -H, -(C₁-C₁₂)-alkyle, -O-(C₁-C₁₂)-alkyle, -O- (C₆-C₂₀) -aryle, - (C₆-C₂₀)-aryle, -S-alkyle, -S-aryle, halogène, -COO-(C₁-C₁₂)-alkyle, -CONH-(C₁-C₁₂)-alkyle, -CO-(C₁-C₁₂)-alkyle, -CO-(C₆-C₂₀)-aryle, -COOH, -OH, -SO₃H, -CN, -NH₂, -N[(C₁-C₁₂)-alkyle]₂ ;
R⁹, R¹⁰, R¹¹, R¹², R¹³, R¹⁴, R¹⁵, R¹⁶, R¹⁷, R¹⁸ sont choisis parmi : -H, - (C₁-C₁₂) -alkyle, -O- (C₁-C₁₂) -alkyle, -O- (C₆-C₂₀)-aryle, -(C₆-C₂₀) -aryle, -S-alkyle, -S-aryle, halogène, -COO- (C₁-C₁₂) -alkyle, -CONH- (C₁-C₁₂)-alkyle, -CO- (C₁-C₁₂) -alkyle, -CO-(C₆-C₂₀)-aryle, -COOH, -OH, -SO₃H, -NH₂, -N [(C₁-C₁₂)-alkyle]₂; les groupes alkyle et aryle mentionnés pouvant être substitués comme suit : (C₁-C₁₂)-alkyle et O-(C₁-C₁₂)-alkyle peuvent à chaque fois être non substitués ou substitués par un ou plusieurs radicaux identiques ou différents, qui sont choisis parmi (C₃-C₁₂)-cycloalkyle, (C₃-C₁₂)-hétérocycloalkyle, (C₆-C₂₀)-aryle, fluor, chlore, cyano, formyle, acyle ou alcoxycarbonyle ;
les groupes - (C₆-C₂₀) -aryle substitués et les groupes - (C₆-C₂₀) -aryl- (C₆-C₂₀) -aryle substitués peuvent présenter, en fonction de leur dimension de cycle, un ou plusieurs substituants ; ces substituants sont choisis, indépendamment les uns des autres, parmi -H, -(C₁-C₁₂)-alkyle, -O-(C₁-C₁₂)-alkyle, -O-(C₆-C₂₀)-aryle, -(C₆-C₂₀)-aryle, -halogène, -COO-(C₁-C₁₂)-alkyle, -CONH-(C₁-C₁₂)-alkyle, -(C₆-C₂₀)-aryl-CON[(C₁-C₁₂)-alkyle]₂, -CO-(C₁-C₁₂)-alkyle, -CO-(C₆-C₂₀)-aryle, -COOH, -OH, -SO₃H, -SO₃Na, -NO₂, -CN, -NH₂, -N[(C₁-C₁₂)-alkyle]₂;
et X et Y sont choisis parmi: -(C₁-C₁₂)-alkyle, -(C₃-C₁₂)-cycloalkyle, -(C₃-C₁₂)-hétérocycloalkyle, -(C₆-C₂₀)-aryle, les groupes alkyle et aryle mentionnés pouvant être substitués comme suit : (C₁-C₁₂) -alkyle et 0- (C₁-C₁₂)-alkyle peuvent à chaque fois être non substitués ou substitués par un ou plusieurs radicaux identiques ou différents, qui sont choisis parmi (C₃-C₁₂)-cycloalkyle, (C₃-C₁₂)-hétérocycloalkyle, (C₆-C₂₀)-aryle, fluor, chlore, cyano, formyle, acyle ou alcoxycarbonyle ;
les groupes -(C₆-C₂₀)-aryle substitués et les groupes - (C₆-C₂₀) -aryl- (C₆-C₂₀) -aryle substitués peuvent présenter, en fonction de leur dimension de cycle, un ou plusieurs substituants ; ces substituants sont choisis, indépendamment les uns des autres, parmi -H, -(C₁-C₁₂)-alkyle, -O-(C₁-C₁₂)-alkyle, -O-(C₆-C₂₀)-aryle, -(C₆-C₂₀)-aryle, -halogène, -COO-(C₁-C₁₂)-alkyle, -CONH-(C₁-C₁₂)-alkyle, -(C₆-C₂₀)-aryl-CON[(C₁-C₁₂)-alkyle]₂, -CO-(C₁-C₁₂)-alkyle, -CO-(C₆-C₂₀)-aryle, -COOH, -OH, -SO₃H, -SO₃Na, -NO₂, -CN, NH₂, -N[(C₁-C₁₂)-alkyle]₂;
et les deux radicaux d'au moins une des quatre paires de radicaux suivantes ne représentent pas le même radical : R¹ et R⁸, R² et R⁷, R³ et R⁶, R⁴ et R⁵.

2. Composé selon la revendication 1, X et Y étant choisis parmi : - (C₁-C₁₂) -alkyle, -(C₃-C₁₂)-cycloalkyle, -(C₆-C₂₀)-aryle.

3. Composé selon l'une quelconque des revendications 1 ou 2, X et Y étant choisis parmi : -(C₁-C₁₂)-alkyle, - (C₃-C₁₂) -cycloalkyle.

4. Composé selon l'une quelconque des revendications 1 à 3, R¹, R², R³, R⁴, R⁵, R⁶, R⁷, R⁸ étant choisis parmi : -H, -(C₁-C₁₂)-alkyle, -O-(C₁-C₁₂)-alkyle, -O-(C₆-C₂₀)-aryle, -(C₆-C₂₀)-aryle, -S-alkyle, -S-aryle, halogène.

5. Composé selon l'une quelconque des revendications 1 à 4, R⁹, R¹⁰, R¹¹, R¹², R¹³, R¹⁴, R¹⁵, R¹⁶, R¹⁷, R¹⁸ étant choisis parmi : -H, (C₁-C₁₂)-alkyle, -O-(C₁-C₁₂)-alkyle, -O-(C₆-C₂₀)-aryle, -(C₆-C₂₀)-aryle, -S-alkyle, -S-aryle, halogène.

6. Composé selon l'une quelconque des revendications 1 à 5, R¹, R², R³, R⁴, R⁵, R⁶, R⁷, R⁸ étant choisis parmi : -H, -(C₁-C₁₂)-alkyle, -O-(C₁-C₁₂)-alkyle, -O-(C₆-C₂₀) -aryle.

7. Composé selon l'une quelconque des revendications 1 à 6, R⁹, R¹⁰, R¹¹, R¹², R¹³, R¹⁴, R¹⁵, R¹⁶, R¹⁷, R¹⁸ étant choisis parmi : -H, - (C₁-C₁₂) -alkyle, -0-(C₁-C₁₂)-alkyle, -O-(C₆-C₂₀) -aryle.

8. Composé selon l'une quelconque des revendications 1 à 7, R¹, R², R³, R⁴, R⁵, R⁶, R⁷, R⁸ étant choisis parmi : -H, (C₁-C₁₂)-alkyle, -O-(C₁-C₁₂)-alkyle.

9. Composé selon l'une quelconque des revendications 1 à 8, R⁹, R¹⁰, R¹¹, R¹², R¹³, R¹⁴, R¹⁵, R¹⁶, R¹⁷, R¹⁸ étant choisis parmi : -H, - (C₁-C₁₂) -alkyle, -O-(C₁-C₁₂)-alkyle.

10. Composé selon l'une quelconque des revendications 1 à 9, qui présente une des structures (2') à (7') suivantes :

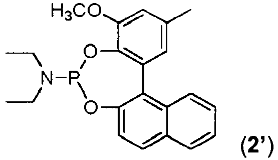

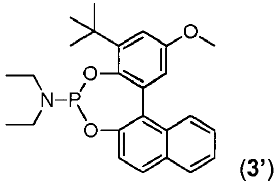

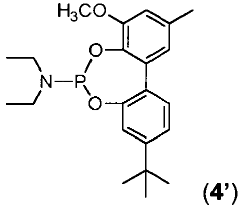

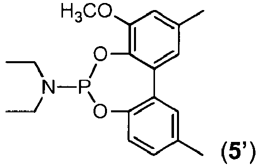

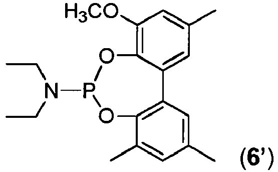

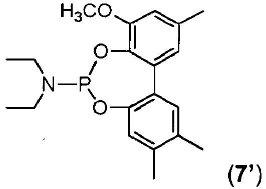

11. Composé selon l'une quelconque des revendications 1 à 10, le composé présentant les structures générales (I).

12. Composé selon l'une quelconque des revendications 1 à 10, le composé présentant les structures générales (II).

13. Utilisation d'un composé selon l'une quelconque des revendications 1 à 12 pour la préparation de monophosphites.

14. Procédé comprenant les étapes de procédé :
a) disposition préalable d'un composé qui présente une des structures générales (III) ou (IV) : 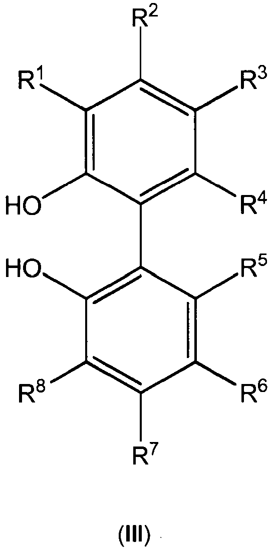 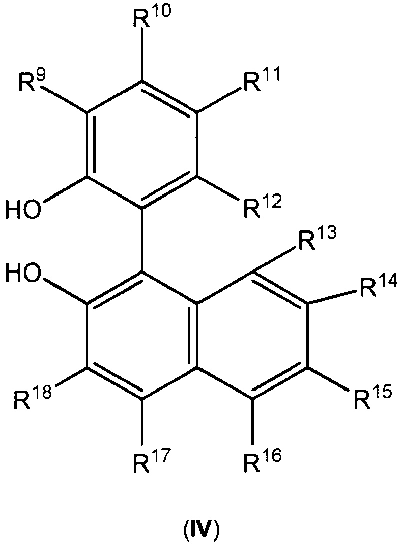
dans lesquelles R¹, R², R³, R⁴, R⁵, R⁶, R⁷, R⁸ sont choisis parmi : -H, (C₁-C₁₂)-alkyle, -O-(C₁-C₁₂)-alkyle, -0- (C₆-C₂₀) -aryle, - (C₆-C₂₀) -aryle, -S-alkyle, -S-aryle, halogène, -COO-(C₁-C₁₂)-alkyle, -CONH-(C₁-C₁₂)-alkyle, -CO-(C₁-C₁₂)-alkyle, -CO-(C₆-C₂₀)-aryle, -COOH, -OH, -SO₃H, -CN, -NH₂, -N[(C₁-C₁₂)-alkyle]₂ ;
R⁹, R¹⁰, R¹¹, R¹², R¹³, R¹⁴, R¹⁵, R¹⁶, R¹⁷, R¹⁸ sont choisis parmi : -H, (C₁-C₁₂)-alkyle, -O-(C₁-C₁₂)-alkyle, -0- (C₆-C₂₀) -aryle, - (C₆-C₂₀) -aryle, -S-alkyle, -S-aryle, halogène, -COO-(C₁-C₁₂)-alkyle, -CONH-(C₁-C₁₂)-alkyle, -CO-(C₁-C₁₂)-alkyle, -CO-(C₆-C₂₀)-aryle, -COOH, -OH, -SO₃H, -NH₂, -N [(C₁-C₁₂) -alkyle] ₂ ; les groupes alkyle et aryle mentionnés pouvant être substitués comme suit : (C₁-C₁₂)-alkyle et O-(C₁-C₁₂)-alkyle peuvent à chaque fois être non substitués ou substitués par un ou plusieurs radicaux identiques ou différents, qui sont choisis parmi (C₃-C₁₂) -cycloalkyle, (C₃-C₁₂)-hétérocycloalkyle, (C₆-C₂₀)-aryle, fluor, chlore, cyano, formyle, acyle ou alcoxycarbonyle ;
les groupes - (C₆-C₂₀) -aryle substitués et les groupes - (C₆-C₂₀) -aryl- (C₆-C₂₀) -aryle substitués peuvent présenter, en fonction de leur dimension de cycle, un ou plusieurs substituants ; ces substituants sont choisis, indépendamment les uns des autres, parmi -H, - (C₁-C₁₂) -alkyle, -O-(C₁-C₁₂)-alkyle, -0- (C₆-C₂₀) -aryle, - (C₆-C₂₀)-aryle, -halogène, -COO-(C₁-C₁₂)-alkyle, -CONH-(C₁-C₁₂)-alkyle, - (C₆-C₂₀) -aryl-CON[(C₁-C₁₂)-alkyle]₂, -CO-(C₁-C₁₂)-alkyle, -CO-(C₆-C₂₀)-aryle, -COOH, -OH, -SO₃H, -SO₃Na, -NO₂, -CN, -NH₂, - N[(C₁-C₁₂)-alkyle]₂;
et les deux radicaux d'au moins une des quatre paires de radicaux suivantes ne représentent pas le même radical : R¹ et R⁸, R² et R⁷, R³ et R⁶, R⁴ et R⁵ ;
b) addition d'un composé qui présente la structure générale (V) : 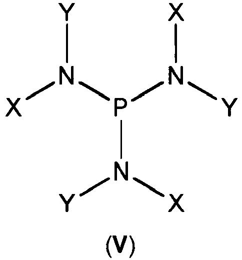 dans laquelle X et Y sont choisis parmi: -(C₁-C₁₂)-alkyle, - (C₃-C₁₂) -cycloalkyle, - (C₃-C₁₂)-hétérocycloalkyle, -(C₆-C₂₀)-aryle ;
c) mélange des composés des étapes de procédé a) et b), (V) étant transformé avec (III) en (I), ou, selon le cas, (V) étant transformé avec (IV) en (II) : 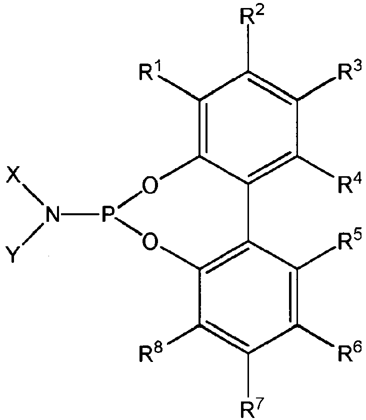 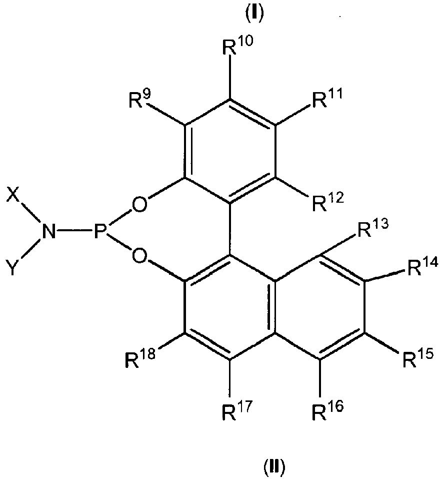 dans lesquelles
R¹, R², R³, R⁴, R⁵, R⁶, R⁷, R⁸ sont choisis parmi : -H, (C₁-C₁₂)-alkyle, -O-(C₁-C₁₂) -alkyle, -O-(C₆-C₂₀) -aryle, - (C₆-C₂₀) -aryle, -S-alkyle, -S-aryle, halogène, -COO- (C₁-C₁₂) -alkyle, -CONH- (C₁-C₁₂)-alkyle, -CO- (C₁-C₁₂) -alkyle, -CO-(C₆-C₂₀)-aryle, -COOH, -OH, -SO₃H, -CN, -NH₂, -N[(C₁-C₁₂)-alkyle]₂ ;
R⁹, R¹⁰, R¹¹, R¹², R¹³, R¹⁴, R¹⁵, R¹⁶, R¹⁷, R¹⁸ sont choisis parmi : -H, (C₁-C₁₂)-alkyle, -O-(C₁-C₁₂)-alkyle, -0- (C₆-C₂₀) -aryle, - (C₆-C₂₀) -aryle, -S-alkyle, -S-aryle, halogène, -COO-(C₁-C₁₂)-alkyle, -CONH-(C₁-C₁₂)-alkyle, -CO-(C₁-C₁₂)-alkyle, -CO-(C₆-C₂₀)-aryle, -COOH, -OH, -SO₃H, -NH₂, -N[(C₁-C₁₂)-alkyle]₂ ;
les groupes alkyle et aryle mentionnés pouvant être substitués comme suit : (C₁-C₁₂)-alkyle et O-(C₁-C₁₂)-alkyle peuvent à chaque fois être non substitués ou substitués par un ou plusieurs radicaux identiques ou différents, qui sont choisis parmi (C₃-C₁₂)-cycloalkyle, (C₃-C₁₂)-hétérocycloalkyle, (C₆-C₂₀)-aryle, fluor, chlore, cyano, formyle, acyle ou alcoxycarbonyle ;
les groupes - (C₆-C₂₀) -aryle substitués et les groupes - (C₆-C₂₀) -aryl- (C₆-C₂₀) -aryle substitués peuvent présenter, en fonction de leur dimension de cycle, un ou plusieurs substituants ; ces substituants sont choisis, indépendamment les uns des autres, parmi -H, -(C₁-C₁₂)-alkyle, -O-(C₁-C₁₂)-alkyle, -O-(C₆-C₂₀)-aryle, -(C₆-C₂₀)-aryle, -halogène, -COO-(C₁-C₁₂)-alkyle, -CONH-(C₁-C₁₂)-alkyle, -(C₆-C₂₀)-aryl-CON[(C₁-C₁₂)-alkyle]₂, -CO-(C₁-C₁₂)-alkyle, -CO-(C₆-C₂₀)-aryle, -COOH, -OH, -SO₃H, -SO₃Na, -NO₂, -CN, -NH₂, -N[(C₁-C₁₂)-alkyle]₂;
et X et Y sont choisis parmi: -(C₁-C₁₂)-alkyle, -(C₃-C₁₂)-cycloalkyle, -(C₃-C₁₂)-hétérocycloalkyle, -(C₆-C₂₀)-aryle, les groupes alkyle et aryle mentionnés pouvant être substitués comme suit : (C₁-C₁₂)-alkyle et O-(C₁-C₁₂)-alkyle peuvent à chaque fois être non substitués ou substitués par un ou plusieurs radicaux identiques ou différents, qui sont choisis parmi (C₃-C₁₂)-cycloalkyle, (C₃-C₁₂)-hétérocycloalkyle, (C₆-C₂₀)-aryle, fluor, chlore, cyano, formyle, acyle ou alcoxycarbonyle ;
les groupes - (C₆-C₂₀) -aryle substitués et les groupes - (C₆-C₂₀) -aryl- (C₆-C₂₀) -aryle substitués peuvent présenter, en fonction de leur dimension de cycle, un ou plusieurs substituants ; ces substituants sont choisis, indépendamment les uns des autres, parmi -H, - (C₁-C₁₂)-alkyle, -O-(C₁-C₁₂) -alkyle, -O-(C₆-C₂₀) -aryle, - (C₆-C₂₀)-aryle, -halogène, -COO-(C₁-C₁₂)-alkyle, -CONH-(C₁-C₁₂)-alkyle, -(C₆-C₂₀)-aryl-CON[(C₁-C₁₂)-alkyle]₂, -CO-(C₁-C₁₂)-alkyle, -CO-(C₆-C₂₀)-aryle, -COOH, -OH, -SO₃H, -SO₃Na, -NO₂, -CN, -NH₂, -N[(C₁-C₁₂)-alkyle]₂;
et les deux radicaux d'au moins une des quatre paires de radicaux suivantes ne représentent pas le même radical :
R¹ et R⁸, R² et R⁷, R³ et R⁶, R⁴ et R⁵.

15. Procédé selon la revendication 14, comprenant l'étape de procédé supplémentaire :
d) addition de naphtol au produit de réaction de l'étape de procédé c).
